# EUROPEAN PATENT SPECIFICATION

(11) **EP 2 350 058 B1**
(45) Date of publication and mention of the grant of the patent: **24.10.2018**
(21) Application number: 09815180.6
(22) Date of filing: 17.09.2009
(51) Int. Cl.: C07D 487/22, C07H 15/26, A61K 31/40, A61K 31/409, A61P 35/00

(54) **NOVEL METHOD AND APPLICATION OF UNSYMMETRICALLY MESO-SUBSTITUTED PORPHYRINS AND CHLORINS FOR PDT**
NEUE VERFAHREN UND ANWENDUNG UNSYMMETRISCH MESO-SUBSTITUIERTER PORPHYRINE UND CHLORINE FÜR DIE PDT
NOUVEAU PROCÉDÉ ET APPLICATION DE PORPHYRINES MÉSO-SUBSTITUÉES DE FAÇON NON SYMÉTRIQUE ET DE CHLORINES POUR THÉRAPIE PHOTODYNAMIQUE

(30) Priority: 18.09.2008 US 98026 P
(43) Date of publication of application: 03.08.2011
(73) Proprietor: biolitec Unternehmensbeteiligungs II AG, 1030 Vienna (AT)
(72) Inventor: WIEHE, Arno, 10777 Berlin (DE); AICHER, Daniel, 12203 Berlin (DE); STARK, Christian, B., W., 04107 Leipzig (DE); GRÄFE, Susanna, 07745 Jena (DE)
(74) Representative: Herrmann, Franz
(86) International application number: PCT/US2009/057283
(87) International publication number: WO 2010/033678

(56) References cited:
- WO-A1-96/16966
- WO-A1-03/055887
- WO-A1-2006/053707
- WO-A2-2004/035590
- DE-A1- 10 146 970
- FR-A1- 2 709 491
- US-A- 5 831 088
- WIEHE A ET AL: "HYDROPHILICITY VS HYDROPHOBICITY-VARYING THE AMPHIPHILIC STRUCTURE OF PORPHYRINS RELATED TO THE PHOTOSENSITIZER M-THPC", JOURNAL OF PORPHYRINS AND PHTHALOCYANINES, BOGNOR REGIS, GB, vol. 5, no. 10, 1 January 2001 (2001-01-01), pages 758-761, XP008038038, ISSN: 1088-4246, DOI: 10.1002/JPP.389

## Description

### Background of the Invention

**Domestic Priority under 35 USC 119(e).** This application claims the benefit of U.S. Provisional Application Serial No. 61/098,026 filed September 18, 2008.

### 1. Field of the invention

The invention relates to the chemistry of biologically active compounds. More particularly to specifically substituted chlorin derivatives that can be used as photosensitizers for a wide range of light irradiation treatments such as photodynamic therapy of cancer, infections and other diseases.

### 2. Invention Disclosure Statement

Photodynamic therapy (PDT) is one of the most promising new techniques now being explored for use in a variety of medical applications (Photodynamic therapy, basic principles and clinical applications. Eds. B. W. Henderson, Th. J. Dougherty, Marcel Dekker, 1992, New York), and particularly is a well-recognized treatment for the destruction of tumors (Photodynamic tumor therapy. 2nd and 3rd generation photosensitizers. Ed. J. G. Moser, Harwood Academic Publishers, 1998, Amsterdam). Photodynamic therapy uses light and a photosensitizer (a dye) to achieve its desired medical effect. A large number of naturally occurring and synthetic dyes have been evaluated as potential photosensitizers for photodynamic therapy. Perhaps the most widely studied class of photosensitizers are the tetrapyrrolic macrocyclic compounds. Among them, especially porphyrins and chlorins have been tested for their PDT efficacy. Porphyrins are macrocyclic compounds with bridges of one carbon atom joining pyrroles to form a characteristic tetrapyrrole ring structure. There are many different classes of porphyrin derivatives including those containing dihydro-pyrrole units. Chlorins, as referred to in the present invention, are porphyrin derivatives containing one dihydro-pyrrole unit whereas bacteriochlorins are characterized by two dihydro-pyrrole units (in general in chlorins one double bond of the aromatic system in *β*-position is absent and in bacteriochlorins two opposite double bonds are absent compared to the porphyrin). As examples of tetrapyrrolic macrocyclic compounds used as photosensitizers, Patent N° US 4,656,186 from Bommer et. al. discloses fluorescent mono, di- or polyamide of an aminocarboxilic acid and tetrapyrrole containing at least three carboxy groups, Patent N° US 7,022,843B1 from MacAlpine et. al. provides *β*, *β'*-dihydroxy meso-substituted chlorin as photosensitizers, and Patent N° US 7,166,719B2 from Pandey et. al. discloses tetrapyrrole compounds containing a fluorinated substituent where the compound is a chlorin or a bacteriochlorin for PDT diagnostic and therapeutic application.

There are several properties that an effective photosensitizer should accomplish. Among them, a desirable characteristic in order to efficiently destroy deep target tissues is a strong absorption at long wavelength. Many current photosensitizers are not efficient enough as they have low absorption in the red region of the spectrum. Chlorins have the advantage that they possess an intense absorption in the red and near-infrared region of the electromagnetic spectrum. As light of longer wavelength penetrates deeper into the tissue, it is thus possible to treat e.g. more expanded tumors, if the PDT is employed for tumor therapy. Chlorins possessing potential for PDT can either be derived from natural sources or from total synthesis.

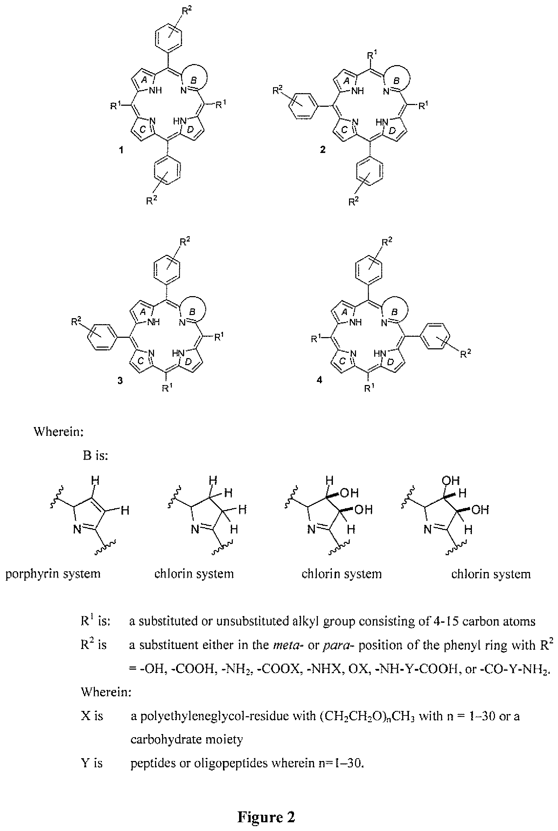

If the chlorins are derived from natural compounds they are usually obtained by derivatizing chlorophylls or bacteriochlorophylls, as for example the photosensitizers derived from chlorophyll a of photosynthetic plants and algae disclosed in Patent N° US 5,330,741. Due to the sensibility of the natural compounds this is often difficult and requires vast resources. So, the synthesis of chlorins by total synthesis is an appealing alternative. Methods to prepare chlorins and bacteriochlorins by total synthesis are known in the art. Generally these compounds are prepared by first synthesizing the porphyrin and then converting the porphyrin system to a chlorin or bacteriochlorin system. This step can e.g. be performed by the reduction with *in situ* generated di-imine or by *cis*-dihydroxylation with osmium tetroxide; multistep reactions leading to *trans*-dihydroxylation are also known (patent EP 00337601B1; patent application WO 09613504A1, patent application WO 00061584A1; C. Brückner, D. Dolphin, 2,3-vic-Dihydroxy-meso-tetraphenylchlorins from the Osmium Tetroxide Oxidation of meso-Tetraphenylporphyrin, Tetrahedron Lett. 1995, 36, 3295-3298; C. Brückner, D. Dolphin, β,β-Dihydroxylation of meso-Tetraphenylchlorins, Tetrahedron Lett. 1995, 36, 9425-9428; H. W. Daniell, S. C. Williams, H. A. Jenkins, C. Brückner, Oxidation of meso-tetra-phenyl-2,3-dihydroxychlorin: simplified synthesis of β,β-dioxochlorins, Tetrahedron Lett. 2003, 44, 4045-4049; F. Rancan, A. Wiehe, M. Nöbel, M. O. Senge, S. A1 Omari, F. Böhm, M. John, B. Röder, Influence of substitutions on asymmetric dihydroxychlorins with regard to intracellular uptake, sub cellular localization and photosensitization in Jurkat cells, J. Photochem. Photobiol. B: Biology 2005, 78, 17-28; I. Laville, T. Figueiredo, B. Loock, S. Pigaglio, Ph. Maillard, D. S. Grierson, D. Carrez, A. Croisy, J. Blais, Synthesis, Cellular Internalization and Photodynamic Activity of Gluco-conjugated Derivatives of Tri and Tetra(meta-hydroxyphenyl)chlorines, Bioorg. Med. Chem. 2003, 11, 1643-1652). Mostly, compounds with four identical substituents in the *meso*-positions have been investigated and tested for their PDT efficacy. One prominent example is Temoporfin which is the active compound in the medicinal product Foscan® which is successfully used in Europe as a medicinal product for the PDT treatment of head and neck cancer. Also, all examples in the abovementioned patent application WO 09613504A1 are compounds with four identical *meso* substituents. The few publications on unsymmetrically tetrakis-*meso*-substituted chlorins derived from total synthesis that exist are of the so-called A₃B-type, i.e. incorporating 3 identical and one different *meso*-substituent (I. Laville, T. Figueiredo, B. Loock, S. Pigaglio, Ph. Maillard, D. S. Grierson, D. Carrez, A. Croisy, J. Blais, Synthesis, Cellular Internalization and Photodynamic Activity of Glucoconjugated Derivatives of Tri and Tetra(meta-hydroxyphenyl)chlorines, Bioorg. Med. Chem. 2003, 11, 1643-1652, F. Rancan, A. Wiehe, M. Nöbel, M. O. Senge, S. A1 Omari, F. Böhm, M. John, B. Röder, Influence of substitutions on asymmetric dihydroxychlorins with regard to intracellular uptake, sub cellular localization and photosensitization in Jurkat cells, J. Photochem. Photobiol. B: Biology 2005, 78, 17-28; J. K. Macalpine, R. Boch, D. Dolphin, Evaluation of tetraphenyl-2,3-dihydroxychlorins as potential photosensitizers, J. Porphyrins Phthalocyanines 2002, 6, 146-155). One reason for using symmetrically substituted porphyrins to convert them into chlorins is that in this case no isomers are formed. If no isomers are formed the resulting compounds are easily characterized and prepared, a key factor for commercial production. If unsymmetrically substituted porphyrins are used to convert them into chlorins different regioisomers are formed which require subsequent separation (not in the case of a *trans*-arrangement of the substituents, cf. Fig. 2). Therefore, the chlorins with a *meso*-A₃B-substitution found in the art are often poorly characterized or are used as an isomeric mixture without separation (e.g. J. K. Macalpine, R. Boch, D. Dolphin, Evaluation of tetraphenyl-2,3-dihydroxychlorins as potential photosensitizers, J. Porphyrins Phthalocyanines 2002, 6, 146-155; I. Laville, T. Figueiredo, B. Loock, S. Pigaglio, Ph. Maillard, D. S. Grierson, D. Carrez, A. Croisy, J. Blais, Synthesis, Cellular Internalization and Photodynamic Activity of Glucoconjugated Derivatives of Tri and Tetra(meta-hydroxyphenyl)chlorines, Bioorg. Med Chem. 2003, 11, 1643-1652). As it is difficult to purify the mixture in order to eliminate the isomers that do not contribute to the PDT effect or enrich the preparation with active compounds, it would be an advantage to find alternative unsymmetrically tetrakis-*meso*-substituted chlorins easily characterized and produced with simple preparation methods. Especially to seize the particular properties of unsymmetrically substituted chlorins, as they might increase the amphiphilicity of the compounds and thus their membrane affinity and PDT efficacy.

Thus, there is a need to enhance the effectiveness of prior art biologically active compounds used as photosensitizers in order to successfully perform a wide range of light irradiation treatments such as photodynamic therapy of cancer, infections and other diseases. Moreover, it is necessary to provide novel methods of preparation and application of unsymmetrically tetrakis-*meso*-substituted chlorins in order to provide enhanced photosensitizers than those available up to date. Thus, PDT efficacy would be increased by taking advantage of unsymmetrically tetrakis-*meso-*substituted chlorins properties, such as strong absorption at long wavelength of the red and near-infrared region of the electromagnetic spectrum for deeper tissue penetration, enhanced selectivity for tumors or other target tissues over healthy surrounding tissues due to its tailored amphiphilicity that increases membrane affinity, and custom-made pharmacokinetic behavior depending on the particular PDT application.

### Objectives and Brief Summary of the Invention

It is an objective of the present invention to provide biologically active compounds that can be used as photosensitizers for a wide range of applications including light irradiation treatments such as photodynamic therapy of cancer, infections and other diseases.

It is a further objective of the present invention to use the chemically stable chlorin derivatives for various medical applications such as photodynamic therapy.

It is another objective of the present invention to provide unsymmetrically tetrakis-*meso*-substituted chlorin structures that can be used in the photodynamic therapy of tumors and other hyperproliferative diseases, dermatological disorders, viral or bacterial infections, opthamological disorders or urological disorders.

It is yet another object of the present invention to provide unsymmetrically tetrakis-*meso*-substituted chlorin structures, as defined in claim 1, that can be used for the fluorescence diagnosis and PDT treatment of a non-tumorous indication such as arthritis and similar inflammatory diseases.

Described is a method to prepare and purify such unsymmetrically tetrakis-*meso*-substituted chlorins and to provide a method for the separation of the isomers formed.

It is still a further object of the present invention to provide highly amphiphilic compounds to be used in the PDT-treatment of tumors, dermatological disorders, viral or bacterial infections, opthamological disorders or urological disorders.

Described is a a method of preparation that can direct the dihydroxylation or reduction of the starting material so that the formation of one isomer is favored.

It is still another objective to provide pharmaceutically acceptable formulations for the biologically active compounds of the present invention such as liposomal formulation to be injected avoiding undesirable effects like precipitation at the injection site or delayed pharmacokinetics of the tetrapyrrole systems.

Briefly stated, the present invention provides Biologically active compounds that can be used as photosensitizers for diagnostic and therapeutic applications, particularly for PDT of cancer, infections and other hyperproliferative diseases, fluorescence diagnosis and PDT treatment of a non-tumorous indication such as arthritis, inflammatory diseases, viral or bacterial infections, dermatological, opthamological or urological disorders as well as providing methods to obtain them in pharmaceutical quality. One embodiment of consists of a method to synthesize a porphyrin with a defined arrangement of *meso*-substituents and then converting this porphyrin system to a chlorin system by dihydroxylation or reduction, and if more than one isomer is formed separate them by chromatography either on normal or reversed phase silica. In another embodiment the substituents on the porphyrin are selected to direct the reduction or dihydroxylation to the chlorin so that a certain isomer is selectively formed. Another embodiment is to provide amphiphilic compounds with a higher membrane affinity and increased PDT-efficacy. In another embodiment a method to reductively cleave the osmate(VI)ester avoiding the use of gaseous H₂S is provided. In another embodiment substituents are identified that via their steric and/or electronic influence direct the dihydroxylation or reduction with diimine so that one isomer is favored. Another embodiment consists of formulate the desired isomer into a liposomal formulation to be injected avoiding undesirable effects like solubility problems at injection or delayed pharmacokinetics of the tetrapyrrole systems

The above and other objects, features and advantages of the present invention will become apparent from the following description read in conjunction with the accompanying drawings.

### Brief Description of Figures

Fig. **1** shows examples of unsymmetrically tetrakis-*meso*-substituted chlorin structures combining two nonpolar (alkyl) and two polar *meso*-substituents that are specially suited for medical applications.
Fig. **2** depicts an embodiment showing the specifically substituted porphyrin derivatives, particularly the chlorin derivatives of types 1, 2, 3 or 4.
Fig. **3** shows an embodiment depicting the arrangement of a porphyrin system to be converted to a chlorin system, where the porphyrin system is of the A₂B₂ type either with a "cis" or a "trans" arrangement of the *meso*-substituents and A is the nonpolar (alkyl) and B the polar substituent.

### Detailed Description of Preferred Embodiments

The present invention provides biologically active compounds that can be used as photosensitizers for a wide range of light irradiation treatments such as photodynamic therapy of cancer, hyperproliferative diseases, dermatological disorders, viral or bacterial infectious diseases, opthamological disorders and/or urological disorders. The alternative photosensitizers provided by the present invention have the advantage that they are easily produced and characterized. Moreover, as the present invention provides methods to tailor amphiphilic compounds for desired PDT applications, target tissue selectivity is increased and thus PDT efficacy. The present invention enhances the effectiveness of prior art biologically active compounds offering a deeper tissue penetration due to their strong absorption at long wavelength of the red and near-infrared region of the electromagnetic spectrum, enhanced selectivity for target tissues over healthy surrounding tissues due to its tailored amphiphilicity and custom-made pharmacokinetic behavior depending on the particular PDT application.

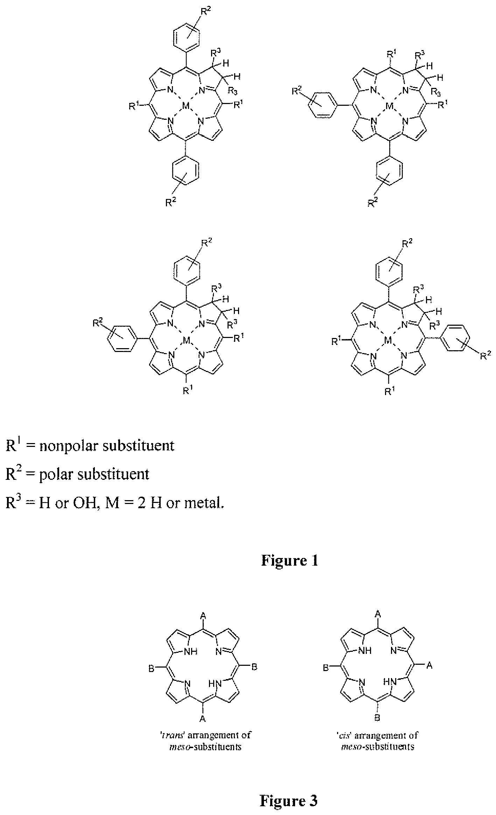

The biologically active compounds of the present invention that can be used for different medical indications, particularly PDT, are unsymmetrically tetrakis-*meso*-substituted chlorin structures. Indeed, it has been unexpectedly been found that chlorins combining two nonpolar (alkyl) and two polar *meso*-substituents in their structure, as illustrated in Fig. 1, are especially suited for such a medical application. Additionally, the novel invention extends its applications as it can be used for fluorescence diagnosis and PDT treatment of a non-tumorous indication such as arthritis and similar inflammatory diseases.

In order to obtain the novel photosensitizers the present description uses the chemically stable porphyrin and chlorin derivatives according to formulae 1, 2, 3, and **4** shown in Fig. 2 and provides methods of preparation and separation of the isomers formed to obtain *meso-*alkyl substituted chlorins, more particularly the unsymmetrically tetrakis-*meso*-substituted chlorin structures that can be used in the photodynamic therapy. With respect to partially *meso*-alkyl substituted chlorins there is in fact only one example in the literature (F. Rancan, A. Wiehe, M. Nöbel, M. O. Senge, S. A1 Omari, F. Böhm, M. John, B. Röder, Influence of substitutions on asymmetric dihydroxychlorins with regard to intracellular uptake, sub cellular localization and photosensitization in Jurkat cells, J. Photochem. Photobiol. B: Biology 2005, 78, 17-28; the compound is also of the *meso*-A₃B-substitution pattern). On the other hand, especially such unsymmetrically substituted chlorins, which are regio-isomerically pure (though in most cases there are still enantiomeric mixtures), could be of great interest as photosensitizers for PDT as such unsymmetric substitution might increase the amphiphilicity of the compounds and thus their membrane affinity and PDT efficacy. In addition, it has surprisingly been found during the investigations related to the present invention, that there are sometimes pronounced differences in PDT-efficacy between different chlorin isomers.

Described is a method to synthesize a porphyrin with a defined arrangement of *meso*-substituents [a porphyrin of the A₂B₂ type, either with a *'cis'* or a *'trans'* arrangement of the *meso*-substituents, as illustrated in Fig. 3, where e.g. A is the nonpolar (alkyl) and B the polar substituent] and then converting this porphyrin system to a chlorin system by dihydroxylation or reduction (as e.g. described in: M. Schroeder, Osmium Tetraoxide Cis Dihydroxylation of Unsaturated Substrates, Chem. Rev. 1980, 80, 187-213; R. Bonnett, R. D. White, U.-J. Winfield, M. C. Berenbaum, Hydroporphyrins of the meso-tetra(hydroxyphenyl)porphyrin series as tumor photosensitizers, Biochem. J. 1989, 261, 277-280). In a last step the isomers (if more than one isomer is formed) are separated by chromatography either on normal or reversed phase silica.

Another embodiment consists of the steps of synthesizing a porphyrin with a defined arrangement of substituents, converting it to the chlorin, separating the isomers as described above and then to formulate the desired isomer into a liposomal formulation.

In yet another embodiment a porphyrin of the *'trans'-*A₂B₂-type is synthesized, converted to the dihydroxychlorin and purified by chromatography.

In yet another embodiment a porphyrin of the '*cis'-*A₂B₂-type is synthesized, converted to the dihydroxychlorin and then the isomers are separated and purified by chromatography.

It has also been found that the substituents on the porphyrin due to their electronic and steric influence can direct the dihydroxylation, thus favoring the formation of one isomer. So, in yet another embodiment the substituents on the porphyrin are selected to direct the reduction or dihydroxylation to the chlorin (examples 3.2 and 3.4) so that a certain isomer is selectively formed.

In a specifically preferred embodiment a porphyrin of the *'trans'*-A₂B₂-type is synthesized, having hexyl chains as substituent A and methoxycarbonyl phenyl residues as substituent B. Then this porphyrin is converted to the dihydroxychlorin and the remaining methylester is hydrolyzed to receive the corresponding carboxylic acid.

Acceptable starting materials for the synthesis of the chlorins which are the subject of the present invention are pyrrole and aldehydes. More specifically, pyrrole and two aldehydes, one alkanal and one aromatic aldehyde are employed for the synthesis of the unsymmetrically substituted porphyrins which are the basis of the synthesis of the corresponding chlorins. Pyrrole and aldehydes are subjected to a condensation reaction. Suitable methods for this condensation have long been known in the art (J. S. Lindsey, I. C. Schreiman, H. C. Hsu, P. C. Kearney and A. M. Marguerettaz, J. Org, Chem. 1987, 52, 827-836). Alternatively, the unsymmetrically substituted porphyrins can also be synthesized using di- or tripyrromethanes and aldehydes, as is also known in the art (C.-H. Lee, J. S. Lindsey, One-Flask Synthesis of Meso-Substituted Dipyrromethanes and Their Application in the Synthesis of Trans-Substituted Porphyrin Building Blocks, Tetrahedron 1994, 50, 11427-11440). After condensation and purification of the desired unsymmetrically substituted porphyrins these are converted to the chlorins. As there is only one example of a tetra-*meso*-substituted chlorin bearing only one alkyl-substituent known in the art, another embodiment provides a method for the preparation of multiply *meso*-alkyl-substituted chlorins via dihydroxylation. The synthesis of *meso-*substituted chlorins bearing alkyl chains is exemplified with examples 3.1-3.4. Furthermore, the use of lipophilic alkyl-substituted porphyrins as substrates for the dihydroxylation is a key feature as it gives access to amphiphilic compounds with a higher membrane affinity and increased PDT-efficacy. In examples 1.1 and 1.2 a series of unsymmetrically substituted porphyrins is synthesized with the objective to obtain porphyrins bearing both hydrophilic and hydrophobic groups.

The dihydroxylation of porphyrins with osmium tetroxide that is known in the art (cf. above Brückner, et al.) uses gaseous H₂S to reductively cleave the osmate(VI)ester. The use of gaseous and toxic H₂S is not favorable for the synthesis of compounds to be used eventually in large scale pharmaceutical preparations. Moreover, the use of hydrogen sulfide leads to impurities, making the chromatographic workup and the separation of the chlorin isomers difficult. Thus, another embodiment provides a simple method for the reductive cleavage of the osmate(VI)ester that avoids the use of gaseous H₂S. Instead, a small amount of a saturated sodium bisulfite solution in water/methanol is used which is added to the reaction mixture. After stirring the mixture overnight the cleavage of the osmate ester to the diol proceeds quantitatively (examples 3.1-3.4). The resulting chlorin mixtures can easily be separated and purified by chromatography.

As the attack of osmium tetroxide or of the diimine can take place on any of the pyrrolic subunits, the reaction of the unsymmetrically substituted porphyrins in the case of the '*cis*'-A₂B₂-type porphyrins leads to the formation of 3 regioisomers, whereas for the *'trans'*-A₂B₂-type only one regioisomer is formed. Therefore, in another embodiment identified are substituents that via their steric and/or electronic influence direct the dihydroxylation or reduction with diimine so that one isomer is favored. In the course of the investigations it turned out that for *'cis'*-A₂B₂-type porphyrins (with A = hexyl) the pyrrolic subunit between hexyl-groups (example 3.2 and 3.4) is preferred for dihydroxylation. This degree of selectivity may be caused by simple steric and/or electronic effects. The structure of the different regioisomers was unequivocally determined by 2D-NMR-spectroscopy (COSY, HMQC and HMBC). Already the ¹H NMR spectra show the influence of the dihydroxylated pyrrolic subunit on the nearby groups in the *meso*-positions. Interestingly, as the dipole moment of the chlorins is affected by the position of the diol also the chromatographic behavior of the compounds in most cases reflects the structure of the corresponding regioisomers.

The use of specifically substituted amphiphilic porphyrin and chlorin derivatives is suitable to be used for photodynamic therapy of cancer and other hyperproliferative diseases and infections. In another embodiment, aimed to obtain such amphiphilic compounds, the methyl ester groups of some porphyrins and dihydroxychlorins were hydrolyzed under basic conditions to provide the corresponding carboxylic acids (example 2 and 4). These acids have an increased solubility in polar solvents, increasing their potential as photosensitizers.

PDT is accomplished by first incorporating the derivatives into a pharmaceutically acceptable application vehicle (e.g. ethanolic solution or liposomal formulation) for delivery of the derivatives to a specific treatment site. After administering the derivatives in the vehicle to a treatment area, sufficient time is allowed so that the porphyrin and chlorine derivatives preferentially accumulate in the diseased tissue. Lastly, the treatment area is irradiated with light of a proper wavelength and sufficient power to activate the porphyrin derivatives to induce necrosis or apoptosis in the cells of said diseased tissue. Thus, one of the main advantages is that convenient pharmaceutical formulations can be created for the biologically active compounds of the present invention such as liposomal formulation to be injected avoiding undesirable effects like precipitation at the injection site or delayed pharmacokinetics of the tetrapyrrole systems. Due to their amphiphilic nature, the chemically stable porphyrin and chlorin derivatives of the present invention can be prepared in various pharmaceutically acceptable and active preparations for different administration methods, e.g. injections. In a specifically preferred embodiment such amphiphilic compounds are formulated into liposomes (example 8.1 and 8.2). This liposomal formulation can then be injected avoiding undesirable effects such as precipitation at the injection site or delayed pharmacokinetics of the tetrapyrrole systems.

Determination of dark toxicity (DT) and photo toxicity (example 6.1) of one specific chlorin derivative of the present invention, 5,15-bis-(4-carboxyphenyl)-10,20-dihexyl-7,8-dihydroxy-7,8-chlorin prepared according to example 4.1, in cell culture experiments with a HT 29 cell line showed the excellent properties of the compounds for use in PDT. Further examples of the good phototoxic properties of the compounds of the present description are illustrated with examples 6.2 and 6.3. The additional examples 6.4 - 6.6 of experiments in the HT 29 cell line are included to illustrate that other dihydroxychlorins which *do not possess* a combination and arrangement of substituents as the one favored in the present invention show a less promising PDT activity.

As another object of the present description is to use the disclosed porphyrin and chlorin derivatives in the diagnosis and treatment of arthritis and similar inflammatory diseases, the data presented in examples 7.1 - 7.4 summarizes the results of the photodynamic treatment of two cell lines especially relevant for arthritis (HIG82 and J774A.1, a rabbit synoviocyte and a mouse macrophage cell line) with a series of compounds of the present description. Again, a negative example (7.5) of a compound not having the favored combination of substituents and lacking the photodynamic activity is also included.

The following examples are presented to provide those of ordinary skill in the art with a full and illustrative disclosure and description of how to make the chlorin derivatives of the invention and show their photodynamic activity and are not intended to limit the scope of what the inventor regards as the invention. Efforts have been made to ensure accuracy with respect to numbers used (e.g. amounts, temperature etc.), but some experimental errors and deviations should be accounted for. Also, best measures have been taken to name the compounds with their systematic IUPAC name, nevertheless the basic reference are the given structural formulas based on the experimental spectroscopic data.

### Examples

All reagents were used as purchased from commercial suppliers. Tetraacetyl-,*β*-D-glucopyranosyloxy-benzaldehyde (I. Laville, S. Pigaglio, J.-C. Blais B. Loock, Ph. Maillard, D. S. Grieson, J. Blais, Biorg. Med. Chem. 2004, 12, 3673-3682) and 5-(4-methoxycarbonylphenyl)-dipyrromethane (B. J. Littler, M. A. Miller, C.-H. Hung, R. W. Wagner, D. F. O'Shea, P. D. Boyle, J. S. Lindsey, J. Org. Chem. 1999, 64, 1391-1396) were prepared according to the literature. Dichloromethane was purified by distillation over K₂CO₃ prior to use. Thin layer chromatography (TLC) was performed using Merck silica gel 60 (without fluorescence indicator) pre-coated on aluminium sheets. Flash chromatography was carried out using Merck silica gel 60, 0.040-0.063 mm (230-400 mesh). ¹H and ¹³C NMR spectra were recorded in CDCl₃, (CD₃)₂CO or (CD₃)₂SO on Bruker AC 250, AC 500 or AMX 500 instruments. Chemical shifts *δ* are given in ppm relative to TMS as internal standard or relative to the resonance of the residual solvent peak, *J* values are given in Hz. Mass spectra were recorded on Varian MAT 771, Varian IonSpec QFT-7 or Agilent 6210 ESI-TOF instruments. Electronic absorption spectra were recorded on a Specord S300 (Analytik Jena) spectrophotometer using dichloromethane or acetone as solvent.

### Example 1

### Reference Example Preparation of unsymmetrical substituted porphyrins

### 1.1 Preparation of 5,15-dihexyl-10,20-bis-(4-methoxycarbonylphenyl)-porphyrin (method A) and 5,10-dihexyl-15,20-bis-(4-methoxycarbonylphenyl)-porphyrin

In a typical experiment, dry dichloromethane (1500 ml) was placed in a three-necked flask equipped with a magnetic stirrer and argon gas inlet. After pyrrole (1.05 ml, 15 mmol), heptanal (1.05 ml, 7.5 mmol) and methyl 4-formylbenzoate (1.23 g, 7.5 mmol) were added, the flask was shielded from ambient light and TFA (1.16 ml, 15 mmol) was added and the reaction mixture was stirred at room temperature for 18 h. Then, DDQ (2.55 g, 11.25 mmol) suspended in dry dichloromethane (100 ml) was added. After further stirring for 1 h, triethylamine (3 ml) was added. To remove polymeric by-products, the reaction mixture was filtered through silica gel. The solvent was evaporated and separation was achieved via flash chromatography with dichloromethane and further purification with dichloromethane/hexane 3:1 (first band) and dichloromethane (second and third band) as eluent. Further purification was achieved by recrystallization from dichloromethane/methanol The first band from the column contained 5,10,15-trihexyl-20-(4-methoxycarbonylphenyl)-porphyrin (203 mg, 12 %), the second band the title compound 5,15-dihexyl-10,20-bis-(4-methoxycarbonylphenyl)-porphyrin (109 mg, 4 %) and the third band the title compound 5,10-dihexyl-15,20-bis-(4-methoxycarbonylphenyl)-porphyrin (199 mg, 7 %).

### 5,15-Dihexyl-10,20-bis-(4-methoxycarbonylphenyl)-porphyrin

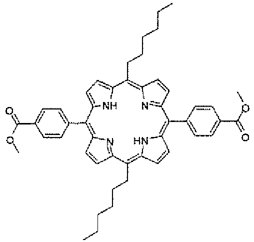

violet microcrystalline solid, mp 238 °C; *λ*ₘₐₓ(CH₂Cl₂)/nm 421 (*ε*/dm³ mol⁻¹ cm⁻¹ 253500), 517 (13400), 553 (7600), 594 (3900) and 650 (5000); *δ*_{H}(250 MHz; CDCl₃) -2.71 (2 H, s, NH), 0.92 (6 H, t, *J* 7.3, 2 × C*H*₃), 1.29-1.53 (8 H, m, 4 × C*H*₂), 1.76 (4 H, m_{c}, 2 × C*H*₂), 2.48 (4 H, m_{c}, 2 × C*H*₂), 4.14 (6 H, s, 2 × OC*H*₃), 4.89 (4 H, t, *J* 7.7, 2 × C*H*₂), 8.27 (4 H, d, *J* 8.2, Ar), 8.45 (4 H, d, *J* 8.2, Ar), 8.79 (4 H, d, *J* 5.0, *β*-H), 9.40 (4 H, d, *J* 5.0, ,*β*-H); *δ*_{C}(63 MHz; CDCl₃) 14.26 (*C*H₃), 22.84 (*C*H₂), 30.37 (CH₂), 32.04 (*C*H₂), 35.49 (*C*H₂), 38.89 (*C*H₂), 52.55 (O*C*H₃), 117.88 (*meso*-C), 120.48 (*meso*-C), 127.93 (Ar), 128.20 (Ar), 129.70 (Ar), 131.46 (Ar), 134.61 (Ar), 147.67 (Ar), 167.53 (*C*O₂CH₃); *m*/*z* (ESI) 747.3904 ([M + H]⁺, C₄₈H₅₁N₄O₄⁺ requires 747.3905).

### 5,10-Dihexyl-15,20-bis-(4-methoxycarbonylphenyl)-porphyrin

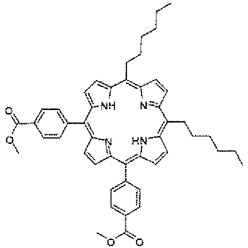

violet microcrystalline solid, mp 131 °C; *λ*ₘₐₓ(CH₂Cl₂)/nm 420 (*ε*/dm³ mol⁻¹ cm⁻¹ 263300), 519 (12000), 553 (7000), 596 (3900) and 652 (4000); *δ*_{H}(250 MHz; CDCl₃) -2.72 (2 H, s, NH), 0.95 (6 H, t, *J* 7.3, 2 x C*H*₃), 1.45 (8 H, m_{c}, 4 x C*H*₂), 1.79 (4 H, m_{c}, 2 x C*H*₂), 2.52 (4 H, m_{c}, 2 x C*H*₂), 4.12 (6 H, s, 2 x OC*H*₃), 4.91 (4 H, t, *J* 8.2, 2 x C*H*₂), 8.24 (4 H, d, *J* 8.2, Ar), 8.43 (4 H, d, *J* 8.2, Ar), 8.69 (2 H, s, *β*-H), 8.78 (2 H, d, *J* 5.0, *β*-H), 9.41 (2 H, d, *J* 5.0, *β*-H), 9.50 (2 H, s, *β*-H); *δ*_{C}(63 MHz; CDCl₃) 14.30 (*C*H₃), 22.89 (*C*H₂), 30.43 (*C*H₂), 32.06 (*C*H₂), 35.86 (*C*H₂), 39.06 (*C*H₂), 52.53 (O*C*H₃), 117.49 (*meso*-C), 120.98 (*meso*-C), 128.05 (Ar), 128.63 (Ar), 129.68 (Ar), 130.88 (Ar), 134.63 (Ar), 147.30 (Ar), 167.51 (*C*O₂CH₃); *mlz* (ESI) 747.3932 ([M + H]⁺, C₄₈H₅₁N₄O₄⁺ requires 747.3905).

### 1.2 Preparation of 5,15-dihexyl-10,20-bis-(4-methoxycarbonylphenyl)-porphyrin (method B)

In a typical experiment, acetonitrile (500 ml) was placed in a three-necked flask equipped with a magnetic stirrer and argon gas inlet. After 5-(4-methoxycarbonylphenyl)-dipyrromethane (714 mg, 2.6 mmol) and heptanal (0.36 ml, 2.6 mmol) were added, the flask was shielded from ambient light and TFA (0.2 ml, 2.6 mmol) was added and the reaction mixture was stirred at room temperature for 18 h. Then, DDQ (860 mg, 3.8 mmol) suspended in acetonitrile (30 ml) was added. After further stirring for 1 h, triethylamine (1 ml) was added. The solvent was evaporated and preliminary purification was achieved via flash chromatography with dichloromethane/methanol 95:5 as eluent and further purification via flash chromatography with dichloromethane/ethylacetate 99:1 as eluent. The title compound 5,15-dihexyl-1 0,20-bis-(4-methoxycarbonylphenyl)-porphyrin was obtained after recrystallization from dichloromethane/methanol (81 mg, 9 %).

### 1.3 Preparation of 5,15-bis-(3-hydroxyphenyl)-10,20-bis-(tridecyl)-porphyrin and 5,10-bis-(3-hydroxyphenyl)-15,20-bis-(tridecyl)-porphyrin

In a typical experiment, dry dichloromethane (1500 ml) was placed in a three-necked flask equipped with a magnetic stirrer and argon gas inlet. After pyrrole (1.05 ml, 15 mmol), tetradecanal (1593 mg, 7.5 mmol) and 3-hydroxybenzaldehyde (916 mg, 7.5 mmol) were added, the flask was shielded from ambient light and TFA (1.16 ml, 15 mmol) was added and the reaction mixture was stirred at room temperature for 4 h. Then, DDQ (2.55 g, 11.25 mmol) suspended in dry dichloromethane (100 ml) was added. After further stirring for 1 h, triethylamine (6 ml) was added. To remove polymeric by-products, the reaction mixture was filtered through silica gel. The solvent was evaporated and separation was achieved via repeated flash chromatography with dichloromethane/ethylacetate 90:10 and 95:5 as eluent. Further purification was achieved by recrystallization from dichloromethane/aqueous methanol The first band from the column contained 5-(3-hydroxyphenyl)-10,15,20-tris-(tridecyl)-porphyrin (68 mg, 4 %), the second band the title compound 5,15-bis-(3-hydroxyphenyl)-10,20-bis-(tridecyl)-porphyrin (52 mg, 2 %) and the third band the title compound 5,10-bis-(3-hydroxyphenyl)-15,20-bis-(tridecyl)-porphyrin (114 mg, 4 %).

### 5,15-Bis-(3-hydroxyphenyl)-10,20-bis-(tridecyl)-porphyrin

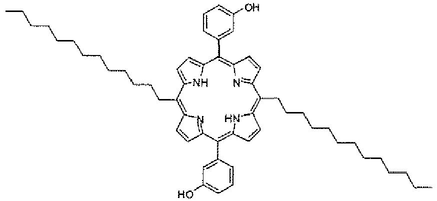

violet microcrystalline solid, mp 133 °C; *δ*_{H}(500 MHz; CDCl₃) -2.70 (2 H, s, NH), 0.87 (6 H, t, *J* 7.0, 2 × C*H*₃), 1.25-1.33 (32 H, m, 16 × C*H*₂), 1.46 (4 H, m_{c}, 2 × C*H*₂), 1.72 (4 H, m_{c}, 2 × C*H*₂), 2.46 (4 H, m_{c}, 2 × C*H*₂), 4.84 (4 H, t, *J* 8.0, 2 × C*H*₂), 7.15-7.17 (2 H, m, Ar), 7.46-7.47 (2 H, m, Ar), 7.54 (2 H, d, *J 7.5,* Ar), 7.73 (2 H, d, *J* 7.5, Ar), 8.83 (4 H, d, *J* 4.7, *β*-H), 9.33 (4 H, d, *J* 4.7, *β*-H); *δ*_{C}(125 MHz; CDCl₃) 14.1 (*C*H₃), 22.7 (*C*H₂), 29.3 (*C*H₂), 29.6 (*C*H₂), 29.7 (*C*H₂), 30.5 (*C*H₂), 31.9 (*C*H₂), 35.2 (*C*H₂), 38.7 (*C*H₂), 114.6 (Ar), 118.2 (*meso*-C), 119.9 (*meso*-C), 121.7 (Ar), 127.5 (Ar), 127.6 (*β*-C), 131.6 (*β*-C), 144.1 (Ar), 153.7 (Ar); *m*/*z* (EI) 858 ([M]⁺, 100%), 689 ([M - C₁₂H₁₅]⁺, 87), 429 ([M]²⁺, 9).

### 5,10-Bis-(3-hydroxyphenyl)-15,20-bis-(tridecyl)-porphyrin

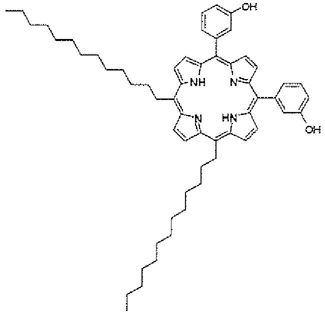

violet microcrystalline solid, mp 113 °C; *δ*_{H}(500 MHz; CDCl₃) -2.68 (2 H, s, NH), 0.88 (6 H, t, *J* 6.9, 2 × C*H*₃), 1.27-1.37 (32 H, m, 16 × C*H*₂), 1.46-1.52 (4 H, m, 2 × C*H*₂), 1.72-1.78 (4 H, m, 2 × C*H*₂), 2.46-2.52 (4 H, m, 2 × C*H*₂), 4.86 (4 H, t, *J* 7.0, 2 × C*H*₂), 6.93-7.09 (2 H, m, Ar), 7.25-7.30 (2 H, m, Ar), 7.38-7.46 (2 H, m, Ar), 7.56-7.65 (2 H, m, Ar), 8.54-8.62 (2 H, m, *β*-H), 8.75-8.79 (2 H, m, *β*-H), 9.30 (2 H, d, *J* 4.8, *β*-H) 9.49 (2 H, s, *β*-H); *δ*_{C}(125 MHz; CDCl₃) 14.1 (*C*H₃), 22.7 (CH₂), 29.7 (*C*H₂), 30.6 (*C*H₂), 31.9 (CH₂), 35.6 (*C*H₂), 38.9 (*C*H₂), 114.5 (Ar), 118.0 (*meso*-C), 120.3 (*meso*-C), 121.6 (Ar), 127.5 (Ar), 127.6 (Ar), 143.5 (Ar), 153.6 (Ar); *mlz* (EI) 858 ([M]⁺, 100%), 689 ([M - C₁₂H₁₅]⁺, 36), 520 ([M - 2C₁₂H₁₅]⁺, 6), 429 ([M]²⁺, 4).

### 1.3 Preparation of 5,10,15-trihexyl-20-(4-methoxycarbonylphenyl)-porphyrin

In a typical experiment, dry dichloromethane (1500 ml) was placed in a three-necked flask equipped with a magnetic stirrer and argon gas inlet. After pyrrole (10.5 ml, 150 mmol), heptanal (15.8 ml, 113 mmol) and methyl 4-formylbenzoate (6.2 g, 38 mmol) were added, the flask was shielded from ambient light and TFA (2.15 ml, 28 mmol) was added and the reaction mixture was stirred at room temperature for 18 h. Then, DDQ (25 g, 110 mmol) suspended in dry dichloromethane (100 ml) was added. After further stirring for 1 h, triethylamine (6 ml) was added. To remove polymeric by-products, the reaction mixture was filtered through silica gel. The solvent was evaporated and separation was achieved via flash chromatography with dichloromethane and further purification with dichloromethane/hexane 3:1. Further purification was achieved by recrystallization from dichloromethane/methanol The first band from the column contained 5,10,15,20-tetrahexyl-porphyrin (930 mg, 5 %), the second band the title compound 5,10,15-trihexyl-20-(4-methoxycarbonylphenyl)-porphyrin (1400 mg, 5 %).

### 5,10,15-Trihexyl-20-(4-methoxycarbonylphenyl)-porphyrin

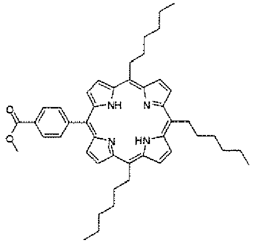

violet microcrystalline solid; *λ*ₘₐₓ(CH₂Cl₂)/nm 418 (*ε*/dm³ mol⁻¹ cm⁻¹ 290300), 519 (14600), 553 (9100), 597 (4200) and 654 (6400); *δ*_{H}(250 MHz; CDCl₃) -2.68 (2 H, s, NH), 0.92-1.06 (9 H, m, 3 × C*H*₃), 1.33-1.60 (12 H, m, 6 × C*H*₂), 1.73-1.88 (6 H, m, 3 × C*H*₂), 2.43-2.60 (6 H, m, 3 × C*H*₂), 4.14 (3 H, s, OC*H*₃), 4.85-4.96 (6 H, m, C*H*₂), 8.25 (2 H, d, *J* 8.2, Ar), 8.43 (2 H, d, *J* 8.2, Ar), 8.73 (2 H, d, *J* 5.5, *β*-H), 9.36 (2 H, d, *J* 5.5, *β*-H), 9.45 (2 H, d, *J* 5.5, *β*-H), 9.48 (2 H, d, *J* 5.5, *β*-H); *δ*_{C}(63 MHz; CDCl₃) 14.30 (*C*H₃), 22.89 (*C*H₂), 22.93 (*C*H₂), 30.39 (*C*H₂), 30.46 (*C*H₂), 32.07 (*C*H₂), 35.37 (*C*H₂), 35.96 (*C*H₂), 38.83 (*C*H₂), 39.04 (*C*H₂), 52.51 (O*C*H₃), 116.54 (*meso*-C), 119.51 (*meso*-C), 120.01 (*meso*-C), 127.95 (Ar), 128.26 (Ar), 129.52 (Ar), 130.84 (Ar), 134.63 (Ar), 147.77 (Ar), 167.58 (*C*O₂CH₃); *m*/*z* (ESI) 697.4467 ([M + H]⁺ C₄₆H₅₇N₄O₂⁺ requires 697.4476).

### Example 2

### Reference Example Preparation of unsymmetrical carboxy-substituted porphyrins

### 2.1 Preparation of 5,15-bis-(4-carboxyphenyl)-10,20-dihexyl-porphyrin

In a typical experiment, a solution of KOH (200 mg, 3.6 mmol) in methanol (1 ml) was added to a stirred solution of 5,15-dihexyl-10,20-bis-(4-methoxycarbonylphenyl)-porphyrin (31 mg, 0.04 mmol) in THF (8 ml) and the reaction mixture was stirred for 2 d. Water (50 ml) and hydrochloric acid were then added until the pH was adjusted to 4-6. The aqueous layer was extracted with dichloromethane (2 x 100 ml) and the organic layer was separated, washed with water until neutral and dried over sodium sulfate. The solvent was evaporated and the title compound 5,15-bis-(4-carboxyphenyl)-10,20-dihexyl-porphyrin was obtained after recrystallization from dichloromethane/aqueous methanol (26 mg, 87 %).

### 5,15-Bis-(4-carboxyphenyl)-10,20-dihexyl-porphyrin

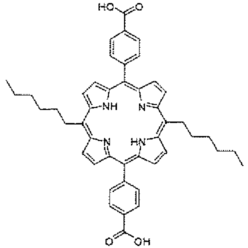

violet microcrystalline solid, *δ*_{H}(250 MHz; (CD₃)₂SO) -2.96 (2 H, s, NH), 0.81 (6 H, t, *J* 7.1, 2 x C*H*₃), 1.15-1.41 (8 H, m, 4 x C*H*₂), 1.60-1.71 (4 H, m, 2 x C*H*₂), 2.25-2.37 (4 H, m, 2 x C*H*₂), 4.81-4.86 (4 H, m, 2 x C*H*₂), 8.21 (4 H, d, *J* 8.1, Ar), 8.33 (4 H, d, *J* 8.1, Ar), 8.71 (4 H, d, *J* 4.8, *β*-H), 9.57 (4 H, d, *J* 4.8, *β*-H); *mlz* (ESI) 719.3621 ([M + H]⁺, C₄₆H₄₇N₄O₄⁺ requires 719.3592).

### 2.2 Preparation of 5,10-bis-(4-carboxyphenyl)-15,20-dihexyl-porphyrin

In a typical experiment, a solution of KOH (200 mg, 3.6 mmol) in methanol (1 ml) was added to a stirred solution of 5,1 0-dihexyl-15,20-bis-(4-methoxycarbonylphenyl)-porphyrin (34 mg, 0.05 mmol) in THF (8 ml) and the reaction mixture was stirred for 2 d. Water (50 ml) and hydrochloric acid were then added until the pH was adjusted to 4-6. The aqueous layer was extracted with dichloromethane (2 x 100 ml) and the organic layer was separated, washed with water until neutral and dried over sodium sulfate. The solvent was evaporated and the title compound 5,10-bis-(4-carboxyphenyl)-15,20-dihexyl-porphyrin was obtained after recrystallization from dichloromethane/aqueous methanol (26 mg, 79 %).

### 5,10-bis-(4-carboxyphenyl)-15,20-dihexyl-porphyrin

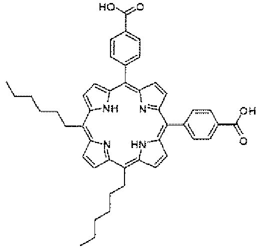

violet microcrystalline solid, *δ*_{H}(250 MHz; (CD₃)₂SO) -2.99 (2 H, s, NH), 0.81 (6 H, t, *J* 7.0, 2 × C*H*₃), 1.19-1.41 (8 H, m, 4 × C*H*₂), 1.60-1.71 (4 H, m, 2 × C*H*₂), 2.25-2.36 (4 H, m, 2 × C*H*₂), 4.78-4.83 (4 H, m, 2 × C*H*₂), 8.18 (4 H, d, *J* 8.0, Ar), 8.30 (4 H, d, *J* 8.0, Ar), 8.63 (2 H, s, *β*-H), 8.69 (2 H, d, *J* 4.7, *β*-H), 9.54 (2 H, d, *J* 4.7, *β*-H), 9.62 (2 H, s, *β*-H); *m*/*z* (ESI) 719.3611 ([M + H]⁺, C₄₆H₄₇N₄O₄⁺ requires 719.3592).

### Example 3

### Preparation of unsymmetrically substituted cis-dihydroxy-chlorins

### 3.1 Preparation of 5,15-dihexyl-7,8-dihydroxy-10,20-bis-(4-methoxy-carbonylphenyl)-7,8-chlorin

In a typical experiment, a solution of osmium tetroxide (40 mg, 0.16 mmol) in dichloromethane/pyridine 30 % (4 ml) was added to a stirred solution of 5,15-dihexyl-10,20-bis-(4-methoxycarbonylphenyl)-porphyrin (90 mg, 0.12 mmol) in dichloromethane/pyridine 30 % (9 ml). After stirring for 3 h a saturated solution of sodium bisulfite in water/methanol 1: 1 (15 ml) was added and the mixture was stirred for 18 h. The reaction mixture was filtered through Celite and dried over sodium sulfate. The solvent was evaporated and the residue was purified by flash chromatography with dichloromethane/ethylacetate 95:5 as eluent, followed by recrystallization from dichloromethane/methanol. The first band from the column contained starting material (20 mg, 22 %) and the second band the title compound 5,15-dihexyl-7,8-dihydroxy-10,20-bis-(4-methoxycarbonylphenyl)-7,8-chlorin (52 mg, 55 %).

### 5,15-Dihexyl-7,8-dihydroxy-10,20-bis-(4-methoxycarbonylphenyl)-7,8-chlorin

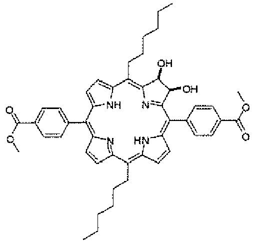

violet microcrystalline solid, mp 124 °C; *λ*ₘₐₓ(CH₂Cl₂)/nm 411 (*ε*/dm³ mol⁻¹ cm⁻¹ 182900), 428 (159800), 524 (15500), 550 (18400), 594 (8200) and 646 (19200); *δ*_{H}(500 MHz; CDCl₃) -1.94 (2 H, br s, NH), 0.89-0.95 (6 H, m, 2 × C*H*₃), 1.31-1.50 (8 H, m, 4 × C*H*₂), 1.67-1.76 (4 H, m, 2 × C*H*₂), 2.15-2.29 (2 H, m, C*H*₂), 2.33-2.41 (2 H, m, C*H*₂), 4.08 (3 H, s, OC*H*₃), 4.11 (3 H, s, OC*H*₃), 4.32-4.39 (1 H, m, H*C*R), 4.48-4.54 (1 H, m, H*C*H), 4.59-4.65 (2 H, m, C*H*₂), 6.22 (1 H, d, *J* 7.1, *β*-H), 6.45 (1 H, d, *J* 7.1, *β*-H)*,* 7.95 (1 H, d, *J* 7.3, Ar), 8.04 (1 H, d, *J* 7.4, Ar), 8.15 (2 H, d, *J* 8.3, Ar), 8.19 (1 H, d, *J* 5.0, *β*-H), 8.32-8.39 (4 H, br m, Ar), 8.42 (1 H, d, *J* 4.6, *β*-H), 8.61 (1 H, d, *J* 4.9, *β*-H), 9.00 (1 H, d, *J* 4.9, *β*-H), 9.06 (1 H, d, *J* 4.6, *β*-H), 9.13 (1 H, d, *J* 5.0, *β*-H); *δ*_{C}(125 MHz; CDCl₃) 14.26 (*C*H₃), 14.30 (*C*H₃), 22.81 (*C*H₂), 22.91 (*C*H₂), 30.33 (*C*H₂), 30.42 (*C*H₂), 31.98 (*C*H₂), 32.07 (*C*H₂), 33.38 (*C*H₂), 35.22 (*C*H₂), 36.76 (*C*H₂), 38.20 (*C*H₂), 52.55 (O*C*H₃), 73.60 (*β*-C), 74.05 (*β*-C), 111.35 (*meso*-C), 113.39 (*meso*-C), 120.54 (*meso*-C), 122.28 (*β*-C), 123.81 (*meso*-C), 124.14 (*β*-C), 124.86 (*β*-C), 127.97 (Ar), 128.62 (*β*-C), 128.73 (Ar), 129.32 (Ar), 129.65 (Ar), 129.75 (*β*-C), 129.81 (*α*-C), 132.35 (Ar), 133.09 (*β*-C), 134.03 (Ar), 134.27 (Ar), 135.72 (*α*-C), 139.04 (*α*-C), 141.43 (*α*-C), 146.84 (Ar), 147.29 (Ar), 151.59 (*α-*C), 153.67 (*α*-C), 159.05 (*α*-C), 162.79 (*α*-C), 167.26 (*C*O₂CH₃), 167.50 (*C*O₂CH₃); *m*/*z* (ESI) 781.3988 ([M + H]⁺, C₄₈H₅₃N₄O₆⁺ requires 781.3960).

### 3.2 Reference Example: Preparation of 5,10-dihexyl-7,8-dihydroxy-15,20-bis-(4-methoxy-carbonylphenyl)-7,8-chlorin, 5,20-dihexyl-7,8-dihydroxy-10,15-bis-(4-methoxycarbonylphenyl)-7,8-chlorin and 5,10-dihexyl-17,18-dihydroxy-15,20-bis-(4-methoxycarbonylphenyl)-17,18-chlorin

In a typical experiment, a solution of osmium tetroxide (100 mg, 0.39 mmol) in dichloromethane/pyridine 30 % (10 ml) was added to a stirred solution of 5,10-dihexyl-15,20-bis-(4-methoxycarbonylphenyl)-porphyrin (226 mg, 0.30 mmol) in dichloromethane/pyridine 30 % (35 ml). After stirring for 20 h a saturated solution of sodium bisulfite in water/methanol 1:1 (40 ml) was added and the mixture was stirred for 18 h. The reaction mixture was filtered through Celite and dried over sodium sulfate. The solvent was evaporated and the residue was purified by flash chromatography with dichloromethane/ethylacetate 90:10 as eluent, followed by recrystallization from dichloromethane/methanol. The first band from the column contained starting material (38 mg, 18 %), the second band the title compound 5,10-dihexyl-7,8-dihydroxy-15,20-bis-(4-methoxycarbonylphenyl)-7,8-chlorin (60 mg, 25 %), the third band the title compound 5,20-dihexyl-7,8-dihydroxy-10,15-bis-(4-methoxycarbonylphenyl)-7,8-chlorin (74 mg, 31 %) and the fourth band contained the title compound 5,10-dihexyl-17,18-dihydroxy-15,20-bis-(4-methoxycarbonylphenyl)-17,18-chlorin (22 mg, 9 %).

### 5,10-Dihexyl-7,8-dihydroxy-15,20-bis-(4-methoxycarbonylphenyl)-7,8-chlorin

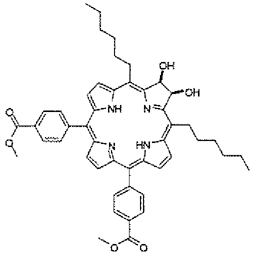

violet microcrystalline solid, mp 171-178 °C; *λ*ₘₐₓ(CH₂Cl₂)/nm 410 (*ε*/dm³ mol⁻¹ cm⁻¹ 346000), 426 (339000), 526 (31100), 553 (38900), 596 (18100) and 648 (39400); *δ*_{H}(500 MHz; CDCl₃) -2.19 (2 H, m, NH), 0.95 (6 H, t, *J* 7.3 Hz, 2 × C*H*₃), 1.36-1.49 (8 H, m, 4 × C*H*₂), 1.66-1.75 (4 H, m, 2 × C*H*₂), 2.09-2.23 (4 H, m, 2 × C*H*₂), 4.06 (6 H, s, 2 × OC*H*₃), 4.25-4.31 (2 H, m, C*H*₂), 4.38-4.44 (2 H, m, C*H*₂), 6.38 (2 H, s, *β-*H), 7.90-7.98 (2 H, br m, Ar), 8.02-8.09 (2 H, br m, Ar), 8.14 (2 H, br s, *β*-H), 8.28-8.30 (4 H, m, Ar), 8.55 (2 H, d, *J* 5.0, *β*-H), 8.95 (2 H, d, *J* 5.0, *β*-H); *δ*_{C}(125 MHz; CDCl₃) 14.31 (*C*H₃), 22.93 (CH₂), 30.43 (*C*H₂), 32.04 (*C*H₂), 33.74 (*C*H₂), 36.86 (*C*H₂), 52.52 (O*C*H₃), 73.89 (*β*-C), 113.47 (*meso*-C), 120.15 (*meso*-C), 122.51 (*β*-C), 127.97 (*β*-C), 129.49 (*β*-C), 132.45 (Ar), 133.88 (Ar), 134.19 (Ar), 134.69 (*α*-C), 140.16 (*α*-C), 146.83 (Ar), 152.33 (*α*-C), 161.12 (*α*-C), 167.45 (*C*O₂CH₃); *m*/*z* (ESI) 781.3993 ([M + H]⁺, C₄₈H₅₃N₄O₆⁺ requires 781.3960).

### 5,20-Dihexyl-7,8-dihydroxy-10,15-bis-(4-methoxycarbonylphenyl)-7,8-chlorin

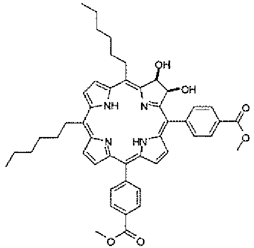

violet microcrystalline solid, mp 128 °C; *λ*ₘₐₓ(CH₂Cl₂)/nm 409 (*ε*/dm³ mol⁻¹ cm⁻¹ 175100), 428 (143800), 524 (14900), 551 (17800), 594 (8400) and 647 (19800); *δ*_{H}(500 MHz; CDCl₃) -2.01 (1 H, s, NH), -1.81 (1 H, s, NH), 0.92-0.98 (6 H, m, 2 × C*H*₃), 1.34-1.52 (8 H, m, 4 × C*H*₂), 1.68-1.77 (4 H, m, 2 × C*H*₂), 2.12-2.20 (1 H, m, H*C*H), 2.21-2.29 (1 H, m, H*C*H), 2.34-2.40 (2 H, m, C*H*₂), 4.05 (3 H, s, OC*H*₃), 4.09 (3 H, s, OC*H*₃), 4.29-4.35 (1 H, m, HC*H*), 4.42-4.49 (1 H, m, H*C*H), 4.53 (2 H, t, *J* 8.3 Hz, C*H*₂), 6.15 (1 H, d, *J* 7.2, *β*-H), 6.37 (1 H, d, *J* 7.2, *β*-H), 7.85 (1 H, d, *J* 7.0, Ar), 7.95 (1 H, d, *J* 7.0, Ar), 8.05-8.10 (1 H, m, Ar), 8.11 (1 H, d, *J* 4.9, *β*-H) 8.16-8.23 (1 H, m, Ar), 8.25-8.38 (4 H, m, Ar), 8.42 (1 H, d, *J* 4.6, *β*-H), 8.47 (1 H, d, *J* 4.9, *β*-H), 8.98 (1 H, d, *J* 5.0, *β*-H), 9.04 (1 H, d, *J* 4.6, *β*-H), 9.16 (1 H, d, *J* 5.0, *β*-H); *δ*_{C}(125 MHz; CDCl₃) 14.28 (*C*H₃), 14.33 (*C*H₃), 22.86 (*C*H₂), 22.95 (*C*H₂), 30.38 (*C*H₂), 30.46 (*C*H₂), 31.98 (*C*H₂), 32.09 (*C*H₂), 33.68 (*C*H₂), 35.48 (*C*H₂), 36.80 (*C*H₂), 38.38 (*C*H₂), 52.52 (O*C*H₃), 73.61 (*β*-C), 73.82 (*β*-C), 111.18 (*meso*-C), 113.40 (*meso*-C), 120.61 (*meso*-C), 122.36 (*β*-C), 123.73 (*meso*-C), 123.95 (*β*-C), 125.72 (*β*-C), 127.61 (*β*-C), 128.08 (Ar), 128.76 (Ar), 129.30 (Ar), 129.65 (Ar), 129.74 (Ar), 130.02 (*β*-C), 132.24 (Ar), 132.71 (*β*-C), 133.95 (Ar), 134.16 (Ar), 134.82 (*α*-C), 134.97 (*α*-C), 139.66 (*α*-C), 140.69 (*α*-C), 146.40 (Ar), 146.90 (Ar), 151.92 (*α*-C), 153.22 (*α*-C), 159.91 (*α*-C), 161.68 (*α*-C), 167.22 (CO₂CH₃), 167.46 (CO₂CH₃); *m*/*z* (EI) 780 ([M]⁺, 21%), 762 ([M - H₂O]⁺, 61), 746 ([M - 2 OH]⁺, 71), 690 ([M - H₂O - C₅H₁₁]⁺, 51), 675 ([M - 2 OH - C₅H₁₁]⁺, 100), 604 ([M - 2 OH - 2 C₅H₁₁]⁺, 30), 390 ([M]²⁺, 8), 381 ([M - H₂O]²⁺, 7), 373 ([M - 2 OH]²⁺, 7); *m*/*z* HRMS (EI) 780.3889 ([M]^{+·}, C₄₈H₅₂N₄O₆^{+·} requires 780.3881).

### 5,10-Dihexyl-17,18-dihydroxy-15,20-bis-(4-methoxycarbonylphenyl)-17,18-chlorin

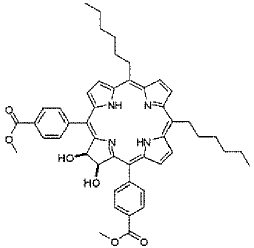

violet microcrystalline solid, mp 121 °C; λₘₐₓ(CH₂Cl₂)/nm 409 (*ε*/dm³ mol⁻¹ cm⁻¹ 163200), 428 (128300), 526sh (16300), 547 (19300), 593 (7700) and 645 (21000); *δ*_{H}(500 MHz; CDCl₃) -1.53 (2 H, s, NH), 0.93 (6 H, t, *J* 7.3 Hz, 2 × C*H*₃), 1.36-1.49 (8 H, m, 4 × C*H*₂), 1.73 (4 H, m_{c}, 2 × C*H*₂), 2.40 (4 H, m_{c}, C*H*₂), 4.05 (6 H, s, 2 × OC*H*₃)*,* 4.58-4.67 (4 H, m, 2 × C*H*₂), 6.10 (2 H, s, *β*-H), 7.89 (2 H, d, *J* 6.3, Ar), 8.08 (2 H, d, *J* 6.3, Ar), 8.19 (2 H, d, *J* 5.0, *β*-H), 8.29 (2 H, d, *J* 6.3, Ar), 8.35 (2 H, d, *J* 6.3, Ar), 9.12 (2 H, d, *J* 5.0 Hz, *β*-H), 9.13 (2 H, s, *β*-H); *δ*_{C}(125 MHz; CDCl₃) 14.28 (*C*H₃), 22.86 (*C*H₂), 30.37 (*C*H₂), 32.00 (*C*H₂), 35.56 (*C*H₂), 38.27 (*C*H₂), 52.47 (O*C*H₃), 73.94 (*β*-C), 111.25 (*meso*-C), 123.83 (*meso*-C), 124.22 (*β*-C), 125.41 (*β*-C), 128.67 (Ar), 129.14 (Ar), 129.63 (Ar), 130.34 (*β*-C), 132.36 (Ar), 134.16 (Ar), 135.14 (*α*-C), 140.19 (*α*-C), 146.53 (Ar), 152.94 (*α*-C), 160.57 (*α*-C), 167.31 (CO₂CH₃); *m*/*z* (ESI) 781.3989 ([M + H]⁺, C₄₈H₅₃N₄O₆⁺ requires 781.3960).

### 3.3 Preparation of 7,8-dihydroxy-5,15-bis-(3-hydroxyphenyl)-10,20-bis-(tridecyl)-7,8-chlorin

In a typical experiment, a solution of osmium tetroxide (36 mg, 0.14 mmol) in dichloromethane/pyridine 30 % (4 ml) was added to a stirred solution of 5,15-bis-(3-hydroxyphenyl)-10,20-bis-(tridecyl)-porphyrin (80 mg, 0.09 mmol) in dichloromethane/pyridine 30 % (6 ml). After stirring for 5 h a saturated solution of sodium bisulfite in water/methanol 1:1 (15 ml) was added and the mixture was stirred for 18 h. The reaction mixture was filtered through Celite and dried over sodium sulfate. The solvent was evaporated and the residue was purified by flash chromatography with dichloromethane/ethylacetate 90:10 as eluent, followed by recrystallization from dichloromethane/aqueous methanol. The first band from the column contained starting material (30 mg, 39 %) and the second band the title compound 7,8-dihydroxy-5,15-bis-(3-hydroxyphenyl)-10,20-bis-(tridecyl)-7,8-chlorin (35 mg, 44 %).

### 7,8-Dihydroxy-5,15-bis-(3-hydroxyphenyl)-10,20-bis-(tridecyl)-7,8-chlorin

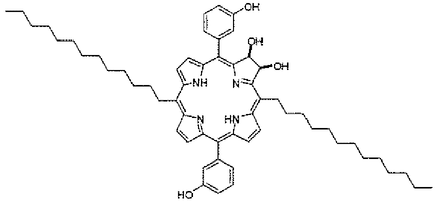

violet microcrystalline solid, mp 111-120 °C; *δ*_{H}(500 MHz; CDCl₃) -1.88 (1 H, s, NH), -1.65 (1 H, s, NH), 0.82-0.85 (6 H, m, 2 × C*H*₃), 1.22-1.34 (32 H, m, 16 × C*H*₂), 1.40-1.49 (4 H, m, 2 × C*H*₂), 1.67-1.74 (4 H, m, 2 × C*H*₂), 2.22-2.40 (4 H, m, 2 × C*H*₂), 4.35-4.41 (1 H, m, HC*H*), 4.61-4.71 (3 H, m, C*H*₂, HC*H*), 6.26-6.33 (1 H, m, *β-*H), 6.50-6.53 (1 H, m, *β*-H), 7.16-7.18 (1 H, m, Ar), 7.25-7.28 (1 H, m, Ar), 7.40-7.63 (6 H, m, Ar), 8.35 (1 H, d, *J* 4.8, *β*-H), 8.52 (1 H, d, *J* 4.9, *β*-H), 8.75 (1 H, d, *J* 4.9, *β-*H), 9.14 (2 H, d, *J* 4.9, *β*-H), 9.32 (1 H, d, *J* 4.8, *β*-H); *δ*_{C}(125 MHz; CDCl₃) 13.5 (*C*H₃), 22.5 (*C*H₂), 28.9 (*C*H₂), 29.1 (*C*H₂), 29.2 (*C*H₂), 29.4 (*C*H₂), 29.5 (*C*H₂), 29.6 (*C*H₂), 29.7 (*C*H₂), 30.2 (*C*H₂), 30.5 (*C*H₂), 31.8 (*C*H₂), 34.5 (*C*H₂), 36.3 (*C*H₂), 38.1 (*C*H₂), 72.9 (*β*-C), 74.5 (*β*-C), 113.0 (*meso*-C), 114.4 (Ar), 114.7 (Ar), 121.4 (Ar), 121.7 (*β*-C), 122.4 (Ar), 124.1 (Ar), 124.4 (*β-*C), 125.8 (Ar), 127.6 (Ar), 128.3 (*β*-C), 129.2 (*β*-C), 132.9 (*β*-C), 134.1 (*α*-C), 135.2 (*α*-C), 139.4 (*α*-C), 140.9 (*α*-C), 143.8 (Ar), 151.8 (*α*-C), 153.4 (*α*-C), 155.8 (Ar); *m*/*z* (EI) 875 ([M - H₂O]^{+·}, 37%), 856 ([M - 2H₂O]^{+·}, 13), 706 ([M - H₂O - C₁₂H₂₅]^{+·}, 6).

### 3.4 Reference Example Preparation of 17,18-dihydroxy-5,10-bis-(3-hydroxyphenyl)-15,20-bis-(tridecyl)-17,18-chlorin, 7,8-dihydroxy-5,20-bis-(3-hydroxyphenyl)-10,15-bis-(tridecyl)-7,8-chlorin and 7,8-dihydroxy-5,10-bis-(3-hydroxyphenyl)-15,20-bis-(tridecyl)-7,8-chlorin

In a typical experiment, a solution of osmium tetroxide (76 mg, 0.30 mmol) in dichloromethane/pyridine 30 % (8 ml) was added to a stirred solution of 5,10-Bis-(3-hydroxyphenyl)-15,20-bis-(tridecyl)-porphyrin (185 mg, 0.22 mmol) in dichloromethane/pyridine 30 % (10 ml). After stirring for 5 h a saturated solution of sodium bisulfite in water/methanol 1:1 (20 ml) was added and the mixture was stirred for 18 h. The reaction mixture was filtered through Celite and dried over sodium sulfate. The solvent was evaporated and the residue was purified by repeated flash chromatography with dichloromethane/ethylacetate 90:10, 40:10 and dichloromethane/methanol 95:5 as eluent, followed by recrystallization from dichloromethane/aqueous methanol. The first band from the column contained starting material (19 mg, 10 %), the second band the title compound 17,18-dihydroxy-5,10-bis-(3-hydroxyphenyl)-15,20-bis-(tridecyl)-17,18-chlorin (49 mg, 25 %), the third band the title compound 7,8-dihydroxy-5,20-bis-(3-hydroxyphenyl)-10,15-bis-(tridecyl)-7,8-chlorin (47 mg, 24 %) and the fourth band contained the title compound 7,8-dihydroxy-5,10-bis-(3-hydroxyphenyl)-15,20-bis-(tridecyl)-7,8-chlorin (25 mg, 13 %).

### 17,18-Dihydroxy-5,10-bis-(3-hydroxyphenyl)-15,20-bis-(tridecyl)-17,18-chlorin

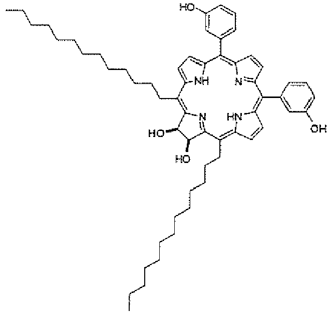

violet microcrystalline solid, mp 103 °C; *δ*_{H}(500 MHz; (CD₃)₂SO) -2.22 (2 H, s, NH), 0.78 (6 H, t, *J* 6.9 2 × C*H*₃), 1.10-1.30 (32 H, m, 16 × C*H*₂), 1.35-1.43 (4 H, m, 2 × C*H*₂), 1.60-1.70 (4 H, m, 2 × C*H*₂), 2.08-2.25 (4 H, m, 2 × C*H*₂), 4.32-4.40 (2 H, m, 2 × HC*H*), 4.53-4.63 (2 H, m, 2 × HC*H*), 6.01 (2 H, s, *β*-OH), 6.44 (2 H, s, *β*-H), 7.15-7.20 (2 H, m, Ar), 7.45-7.54 (6 H, m, Ar), 8.39 (2 H, s, *β*-H), 8.72 (2 H, d, J4.7, *β*-H), 9.19 (2 H, d, *J* 4.7, *β*-H), 9.79 (2 H, s, Ar-OH); *δ*_{C}(125 MHz; (CD₃)₂SO) 14.4 (*C*H₃), 22.6 (*C*H₂), 29.2 (*C*H₂), 29.5 (*C*H₂), 29.6 (*C*H₂), 30.5 (*C*H₂), 31.8 (*C*H₂), 33.4 (*C*H₂), 36.6 (*C*H₂), 73.3 (*β*-C), 113.4 (*meso*-C), 115.4 (Ar), 120.8 (*meso*-C), 122.9 (*β*-C), 125.6 (Ar), 128.3 (Ar), 128.3 (*β*-C), 132.5 (*β*-C), 134.5 (*α*-C), 140.0 (*α*-C), 143.1 (Ar), 152.1 (*α*-C), 156.4 (Ar), 164.0 (*α-*C); *m*/*z* (EI) 892 ([M^{+·}, 5%), 874 ([M - H₂O]^{+·}, 100), 858 ([M - 2 OH)]^{+·}, 31) 705 ([M - H₂O - C₁₂H₂₅]^{+·}, 14).

### 7,8-Dihydroxy-5,20-bis-(3-hydroxyphenyl)-10,15-bis-(tridecyl)-7,8-chlorin

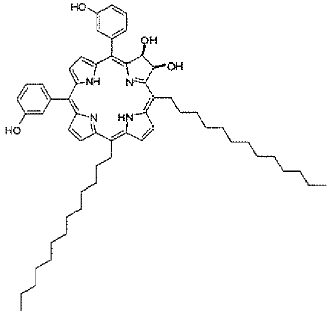

violet microcrystalline solid, mp 203-205 °C; *δ*_{H}(500 MHz; (CD₃)₂SO) -2.02 (1 H, s, NH), -1.92 (1 H, s, NH), 0.78-0.82 (6 H, m, 2 × C*H*₃), 1.12-1.46 (36 H, m, 18 × C*H*₂), 1.59-1.71 (4 H, m, 2 × C*H*₂), 2.10-2.25 (2 H, m, C*H*₂), 2.26-2.32 (2 H, m, C*H*₂), 4.35-4.41 (1 H, m, HC*H*), 4.60-4.66 (1 H, m, HC*H*), 4.67-4.73 (2 H, m, C*H*₂), 5.31 (1 H, s, *β*-OH), 5.81(1 H, s, *β*-OH), 6.05-6.11 (1 H, m, *β*-H), 6.38-6.44 (1 H, m, *β*-H), 7.05-7.07 (1 H, m, Ar), 7.14-7.19 (1 H, m, Ar), 7.25-7.56 (6 H, m, Ar), 8.23 (1 H, d, *J* 4.8, *β*-H), 8.43 (1 H, d, *J* 4.6, *β*-H), 8.56 (1 H, d, *J* 4.9, *β*-H), 9.17 (1 H, d, *J* 4.6, *β*-H), 9.25 (1 H, d, *J* 4.8, *β*-H), 9.54 (1 H, d, *J* 4.9, *β*-H); *δ*_{C}(125 MHz; (CD₃)₂SO) 14.4 (*C*H₃), 14.5(*C*H₃), 22.6 (*C*H₂), 29.2 (*C*H₂), 29.5 (*C*H₂), 29.6 (*C*H₂), 29.7 (*C*H₂), 31.8 (*C*H₂), 38.5 (*C*H₂), 73.3 (*β*-C), 74.6 (*β*-C), 122.8 (*β*-C), 124.8 (*β*-C), 126.5 (*β*-C), 128.3 (*β*-C), 130.6 (*β*-C), 132.8 (*β*-C), 134.6 (*α*-C), 143.1 (Ar), 151.7 (*α*-C), 152.6 (*α*-C), 164.1 (*α*-C); *m*/*z* (EI) 874 ([M - H₂O]^{+·}, 20%), 705 ([M - H₂O - C₁₂H₂₅]^{+·}, 9).

### 7,8-Dihydroxy-5,10-bis-(3-hydroxyphenyl)-15,20-bis-(tridecyl)-7,8-chlorin

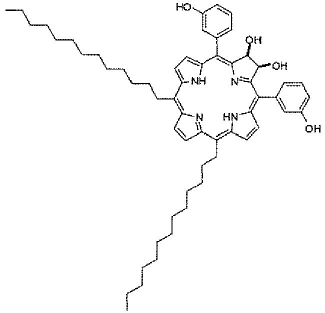

violet microcrystalline solid, mp 137-141 °C; *δ*_{H}(500 MHz; (CD₃)₂CO) -1.56 (2 H, s, NH), 0.83-0.86 (6 H, m, 2 × C*H*₃), 1.24-1.35 (32 H, m, 16 × C*H*₂), 1.45-1.51 (4 H, m, 2 × C*H*₂), 1.72-1.78 (4 H, m, 2 × C*H*₂), 2.42 (4 H, m_{c}, 2 × C*H*₂), 4.70-4.80 (4 H, m, 2 × C*H*₂), 6.18-6.28 (2 H, m, *β*-H), 7.12-7.15 (2 H, m, Ar), 7.30-7.63 (6 H, m, Ar), 8.37 (2 H, d, *J* 4.8, *β*-H), 8.59 (2 H, s, Ar-OH), 9.26 (2 H, s, *β*-H), 9.36 (2 H, d, *J* 4.8, *β-*H); *δ*_{C}(125 MHz; (CD₃)₂CO) 13.5 (*C*H₃), 22.5 (*C*H₂), 28.9 (*C*H₂), 29.1 (*C*H₂), 29.2 (*C*H₂), 29.3 (*C*H₂), 29.4 (*C*H₂), 29.5 (*C*H₂), 29.6 (*C*H₂), 30.2 (*C*H₂), 31.8 (*C*H₂), 34.9 (*C*H₂), 38.2 (*C*H₂), 73.9 (*β*-C), 112.7 (*meso*-C), 122.7 (*meso*-C), 124.1 (*β*-C), 125.1 (*β*-C), 130.1 (*β*-C), 134.8 (*α*-C), 140.5 (*α*-C), 143.1 (Ar), 152.6 (*α*-C), 162.6 (*α*-C); *m*/*z* (EI) 874 ([M - H₂O]^{+·}, 1%), 705 ([M - H₂O - C₁₂H₂₅]^{+·}, 1).

### 3.5 Reference Example Preparation of 5,10,15-trihexyl-7,8-dihydroxy-20-(4-methoxycarbonylphenyl)-7,8-chlorin and 5,10,15-trihexyl-17,18-dihydroxy-20-(4-methoxycarbonylphenyl)-17,18-chlorin

In a typical experiment, a solution of osmium tetroxide (100 mg, 0.39 mmol) in dichloromethane/pyridine 30 % (10 ml) was added to a stirred solution of 5,10,15-trihexyl-20-(4-methoxycarbonylphenyl)-porphyrin (212 mg, 0.30 mmol) in dichloromethane/pyridine 30 % (45 ml). After stirring for 13 days a saturated solution of sodium bisulfite in water/methanol 1:1 (40 ml) was added and the mixture was stirred for 18 h. The reaction mixture was filtered through Celite and dried over sodium sulfate. The solvent was evaporated and the residue was purified by repeated flash chromatography with dichloromethane/ethylacetate 95:5 as eluent, followed by recrystallization from dichloromethane/aqueous methanol. The first band from the column contained starting material (103 mg, 49 %), the second band the title compound 5,10,15-trihexyl-7,8-dihydroxy-20-(4-methoxycarbonylphenyl)-7,8-chlorin (48 mg, 22 %) and the third band the title compound 5,10,15-trihexyl-17,18-dihydroxy-20-(4-methoxycarbonylphenyl)-17,18-chlorin (34 mg, 15 %).

### 5,10,15-Trihexyl-7,8-dihydroxy-20-(4-methoxycarbonylphenyl)-7,8-chlorin

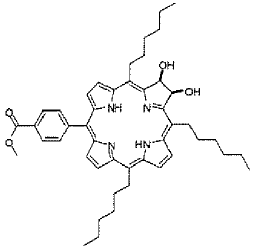

violet microcrystalline solid, mp 109-111 °C; *λ*ₘₐₓ(CH₂Cl₂)/nm 410 (*ε*/dm³ mol cm⁻¹ 146100), 430 (121700), 527 (12400), 556 (16300), 596 (7400), and 649 (14900); *δ*_{H}(500 MHz; CDCl₃) -2.15 (2 H, br s, NH), 0.90-0.94 (6 H, m, 2 × C*H*₃), 0.95 (3 H, t, *J* 7.0, C*H*₃), 1.31-1.47 (12 H, m, 6 × C*H*₂), 1.59-1.71 (6 H, m, 3 × C*H*₂), 1.97-2.12 (4 H, m, 2 × C*H*₂), 2.17-2.29 (2 H, m, C*H*₂), 4.02-4.37 (9 H, m, 3 × C*H*₂, OC*H*₃)*,* 6.14-6.17 (2 H, m, *β*-H), 8.11-8.22 (2 H, br m, Ar), 8.38 (2 H, d, *J* 8.3 Hz, Ar), 8.42 (1 H, d, *J* 4.4, *β*-H), 8.59 (1 H, d, *J* 4.8, *β*-H), 8.61 (1 H, d, *J* 4.7, *β*-H), 8.73 (1 H, d, *J* 4.7, *β*-H), 8.94 (1 H, d, *J* 4.8, *β*-H), 8.99 (1 H, d, *J* 4.4, *β*-H); *δ*_{C}(125 MHz; CDCl₃) 14.29 (*C*H₃), 14.33 (*C*H₃), 22.86 (*C*H₂), 22.89 (*C*H₂), 22.94 (*C*H₂), 30.33 (*C*H₂), 30.34 (*C*H₂), 30.38 (*C*H₂), 31.95 (*C*H₂), 32.01 (*C*H₂), 32.03 (*C*H₂), 33.24 (*C*H₂), 33.59 (*C*H₂), 36.57 (CH₂), 36.68 (*C*H₂), 38.09 (*C*H₂), 52.56 (O*C*H₃), 73.33 (*β*-C), 73.83 (*β-*C), 112.20 (*meso*-C), 112.24 (*meso*-C), 119.32 (*meso*-C), 121.83 (*β*-C), 122.24 (*meso-*C), 122.43 (*β*-C), 124.87 (*β*-C), 127.97 (Ar), 128.01 (*β*-C), 129.52 (Ar), 129.67 (*β*-C), 132.61 (*β*-C), 134.19 (Ar), 134.61 (*α*-C), 139.42 (*α*-C), 140.38 (*α*-C), 147.34 (Ar), 151.53 (*α*-C), 152.94 (*α*-C), 159.70 (*α*-C), 161.12 (*α*-C), 167.56 (*C*O₂CH₃); *m*/*z* (ESI) 731.4485 ([M + H]⁺ C₄₆H₅₉N₄O₄⁺ requires 731.4531).

### 5,10,15-Trihexyl-17,18-dihydroxy-20-(4-methoxycarbonylphenyl)-17,18-chlorin

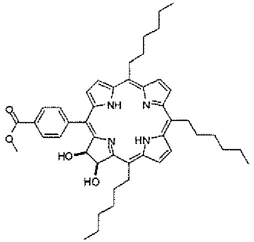

violet microcrystalline solid; *λ*ₘₐₓ(CH₂Cl₂)/nm 409 (*ε*/dm³ mol⁻¹ cm⁻¹ 209900), 430 (167300), 527 (17900), 554 (22200), 595 (10300) and 647 (19800); *δ*_{H}(500 MHz; CDCl₃) -1.86 (2 H, br s, NH), 0.92-0.97 (9 H, m, 3 × C*H*₃), 1.34-1.51 (12 H, m, 6 × C*H*₂), 1.67-1.76 (6 H, m, 3 × C*H*₂), 2.07-2.29 (2 H, m, C*H*₂), 2.34-2.42 (4 H, m, 2 × C*H*₂), 4.06 (3 H, s, OC*H*₃), 4.21-4.28 (1 H, m, HC*H*), 4.34-4.40 (1 H, m, HC*H*), 4.52-4.59 (4 H, m, 2 × C*H*₂), 6.01 (1 H, d, *J* 7.2, *β*-H), 6.26 (1 H, d, *J* 7.2, *β*-H), 7.79-7.90 (2 H, br m, Ar), 8.08 (1 H, d, *J* 5.0, *β*-H), 8.23-8.33 (2 H, br m, Ar), 8.91 (1 H, d, *J* 5.0, *β*-H), 9.05 (1 H, d, *J* 5.0, *β*-H), 9.09 (2 H, s, *β*-H), 9.13 (1 H, d, *J* 7.2, *β-*H); *δ*_{C}(125 MHz; CDCl₃) 14.30 (*C*H₃), 14.33 (*C*H₃), 22.87 (*C*H₂), 22.91 (*C*H₂), 22.95 (*C*H₂), 30.37 (*C*H₂), 30.42 (*C*H₂), 32.00 (*C*H₂), 32.01 (*C*H₂), 32.09 (*C*H₂), 33.34 (*C*H₂), 35.30 (*C*H₂), 35.58 (*C*H₂), 36.61 (*C*H₂), 38.15 (*C*H₂), 38.33 (*C*H₂), 52.51 (O*C*H₃), 73.52 (*β*-C), 73.85 (*β*-C), 110.27 (*meso*-C), 112.26 (*meso*-C), 121.92 (*β*-C), 122.91 (*meso*-C), 122.93 (*meso*-C), 123.60 (*β*-C), 124.83 (*β*-C), 125.67 (*β*-C), 128.65 (Ar), 129.18 (Ar), 129.54 (Ar), 129.92 (*β*-C), 130.21 (*β*-C), 132.34 (Ar), 133.90 (Ar), 134.41 (*α*-C), 135.08 (*α*-C), 139.21 (*α*-C), 140.87 (*α*-C), 146.75 (Ar), 152.35 (*α*-C), 152.76 (*α*-C), 158.99 (*α*-C), 161.59 (*α*-C), 167.30 (*C*O₂CH₃); *m*/*z* (EI) 730 ([M]⁺, 29%), 712 ([M - H₂O]⁺, 100), 697 ([M - 2 OH]⁺, 42), 641 ([M - H₂O - C₅H₁₁]⁺, 57), 623 ([M - 2 OH - C₅H₁₁]⁺, 71); *m*/*z* HRMS (EI) 730.4454 ([M]^{+·}, C₄₆H₅₈N₄O₄^{+·} requires 730.4453).

### Example 4

### Preparation of unsymmetrical carboxy-substituted chlorins

### 4.1 Preparation of 5,15-bis-(4-carboxyphenyl)-10,20-dihexyl-7,8-dihydroxy-7,8-chlorin

In a typical experiment, a solution of KOH (100 mg, 1.8 mmol) in methanol (1 ml) was added to a stirred solution of 5,15-dihexyl-7,8-dihydroxy-10,20-bis-(4-methoxycarbonylphenyl)-7,8-chlorin (20 mg, 0.016 mmol) in THF (3 ml) and the reaction mixture was stirred for 2 d. Water (50 ml) and hydrochloric acid were then added until the pH was adjusted to 4-6. The aqueous layer was extracted with dichloromethane (2 x 100 ml) and the organic layer was separated, washed with water until neutral and dried over sodium sulfate. The solvent was evaporated and the title compound 5,15-bis-(4-carboxyphenyl)-10,20-dihexyl-7,8-dihydroxy-7,8-chlorin was obtained after recrystallization from dichloromethane/aqueous methanol (18 mg, 92 %).

### 5,15-Bis-(4-carboxyphenyl)-10,20-dihexyl-7,8-dihydroxy-7,8-chlorin

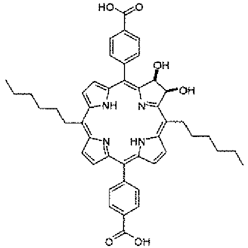

violet microcrystalline solid, mp > 300 °C; *λ*ₘₐₓ((CH₃)₂CO)/nm 407 (*ε*/dm³ mol⁻¹ cm⁻¹ 154700), 427 (130300), 521 (13600), 548 (15300), 594 (6500) and 646 (17600); *δ*_{H}(500 MHz; (CD₃)₂SO) -2.05 (1 H, s, NH), -1.85 (1 H, s, NH), 0.84 (3 H, t, *J* 7.3 Hz, C*H*₃), 0.91 (3 H, t, *J* 7.3, C*H*₃), 1.24-1.48 (8 H, m, 4 × C*H*₂), 1.64-1.67 (2 H, m, C*H*₂), 1.69-1.75 (2 H, m, C*H*₂), 2.12-2.23 (2 H, m, C*H*₂), 2.25-2.31 (2 H, m, C*H*₂), 4.35-4.41 (1 H, m, HC*H*), 4.57-4.64 (1 H, m, HC*H*), 4.66-4.72 (2 H, m, C*H*₂), 5.40 (1 H, br s, OH), 5.86 (1 H, br s, OH), 6.09 (1 H, d, *J* 6.9, *β*-H), 6.40 (1 H, d, *J* 6.9, *β*-H), 8.02-8.35 (10 H, m, 8 × Ar, 2 × *β*-H), 8.64 (1 H, d, *J* 5.0, *β*-H), 9.19 (1 H, d, *J* 4.7, *β*-H), 9.24 (1 H, d, *J* 5.0, *β*-H), 9.45 (1 H, d, *J* 5.2, *β*-H); *δ*_{C}(125 MHz; (CD₃)₂SO) 13.93 (*C*H₃), 14.02 (*C*H₃), 22.10 (*C*H₂), 22.23 (*C*H₂), 29.30 (*C*H₂), 29.67 (*C*H₂), 31.27 (*C*H₂), 31.36 (*C*H₂), 33.88 (*C*H₂), 111.99 (*meso*-C), 113.10 (*meso*-C), 119.32 (*meso-*C), 122.64 (*β*-C), 123.87 (*β*-C), 127.79 (Ar), 128.23 (*β*-C), 132.40 (*β*-C), 133.59 (Ar), 146.10 (Ar), 146.46 (Ar) 167.50 (*C*O₂H), 167.67 (*C*O₂H); *m*/*z* (ESI) 753.3664 ([M + H]⁺, C₄₆H₄₉N₄O₆⁺ requires 753.3647).

### 4.2 Reference Example Preparation of 5,20-bis-(4-carboxyphenyl)-10,15-dihexyl-7,8-dihydroxy-7,8-chlorin

In a typical experiment, a solution of KOH (200 mg, 3.6 mmol) in methanol (1 ml) was added to a stirred solution of 5,20-dihexyl-7,8-dihydroxy-10,15-bis-(4-methoxycarbonylphenyl)-7,8-chlorin (35 mg, 0.045 mmol) in THF (3 ml) and the reaction mixture was stirred for 2 d. Water (50 ml) and hydrochloric acid were then added until the pH was adjusted to 4-6. The aqueous layer was extracted with dichloromethane (2 x 100 ml) and the organic layer was separated, washed with water until neutral and dried over sodium sulfate. The solvent was evaporated and the title compound 5,20-bis-(4-carboxyphenyl)-10,15-dihexyl-7,8-dihydroxy-7,8-chlorin was obtained after recrystallization from dichloromethane/aqueous methanol (31 mg, 91 %).

### 5,20-Bis-(4-carboxyphenyl)-10,15-dihexyl-7,8-dihydroxy-7,8-chlorin

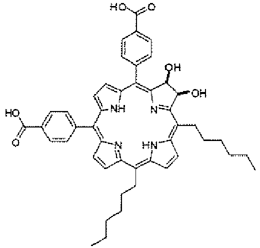

violet microcrystalline solid, mp > 300 °C; *λ*ₘₐₓ((CH₃)₂CO)/nm 407 (*ε*/dm³ mol⁻¹ cm⁻¹ 122800), 427 (96000), 523 (10100), 548 (11500), 595 (4600) and 646 (13900); *δ*_{H}(500 MHz; (CD₃)₂CO) -1.88 (1 H, s, NH), -1.74 (1 H, s, NH), 0.91 (3 H, t, *J* 7.3 Hz, C*H*₃), 0.96 (3 H, t, *J* 7.3, C*H*₃), 1.34-1.56 (8 H, m, 4 × C*H*₂), 1.75-1.85 (4 H, m, 2 × C*H*₂), 2.28-2.46 (4 H, m, 2 × C*H*₂), 4.44-4.50 (1 H, m, HC*H*), 4.65-4.72 (1 H, m, HC*H*), 4.77 (2 H, m_{c}, C*H*₂), 6.33 (1 H, d, *J* 7.0, *β*-H), 6.53 (1 H, d, *J* 7.0, *β*-H), 8.24 (1 H, d, *J* 4.9, *β*-H), 8.00-8.40 (8 H, br m, Ar), 8.42 (1 H, d, *J* 4.5, *β*-H), 8.56 (1 H, d, *J* 4.9, *β*-H), 9.21 (1 H, d, *J* 4.5, *β*-H), 9.30 (1 H, d, *J* 5.0, *β*-H), 9.56 (1 H, d, *J* 5.0, *β*-H); *δ*_{C}(125 MHz; (CD₃)₂SO) 14.00 (*C*H₃), 14.10 (*C*H₃), 22.19 (*C*H₂), 22.33 (*C*H₂), 29.38 (*C*H₂), 29.76 (*C*H₂), 31.33 (*C*H₂), 31.44 (*C*H₂), 32.86 (*C*H₂), 34.39 (*C*H₂), 36.16 (*C*H₂), 38.12 (*C*H₂), 72.91 (*β*-C), 73.13 (*β*-C), 111.93 (*meso*-C), 113.17 *(meso-C),* 119.45 *(meso-C),* 122.66 (*β*-C), 124.10 (*β*-C), 126.43 (*β*-C), 127.44 (*β*-C), 127.92 (Ar), 129.52 (Ar), 130.20 (*β*-C), 132.11 (*β*-C), 133.76 (*α*-C), 133.87 (Ar), 134.29 (*α-*C), 139.06 (*α*-C), 140.04 (*α*-C), 145.84 (Ar), 146.22 (Ar), 150.78 (*α*-C), 152.25 (*α-*C), 163.07 (*α*-C), 163.91 (*α*-C), 167.53 (CO₂H), 167.71 (CO₂H); *m*/*z* (ESI) 753.3637 ([M+ H]⁺, C₄₆H₄₉N₄O₆⁺ requires 753.3647).

### 4.3 Reference Example Preparation of 5-(4-carboxyphenyl)-10,15,20-trihexyl-17,18-dihydroxy-17,18-chlorin

In a typical experiment, a solution of KOH (100 mg, 1.8 mmol) in methanol (0.5 ml) was added to a stirred solution of 5,10,15-trihexyl-7,8-dihydroxy-20-(4-methoxycarbonylphenyl)-7,8-chlorin (12 mg, 0.016 mmol) in THF (2 ml) and the reaction mixture was stirred for 2 d. Water (50 ml) and hydrochloric acid were then added until the pH was adjusted to 4-6. The aqueous layer was extracted with dichloromethane (2 x 100 ml) and the organic layer was separated, washed with water until neutral and dried over sodium sulfate. The solvent was evaporated and the title compound 5-(4-carboxyphenyl)-10,15,20-trihexyl-17,18-dihydroxy-17, 18-chlorin was obtained after recrystallization from dichloromethane/aqueous methanol (10 mg, 87 %).

### 5-(4-Carboxyphenyl)-10,15,20-trihexyl-17,18-dihydroxy-17,18-chlorin

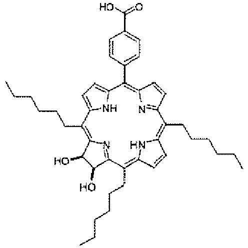

violet microcrystalline solid, mp 134 °C; *λ*ₘₐₓ((CH₃)₂CO)/nm 408 (*ε*/dm³ mol⁻¹ cm⁻¹ 88600), 428 (73300), 526 (8500), 553 (9200), 595 (4300) and 649 (7400); *δ*_{H}(500 MHz; (CD₃)₂CO) -1.98 (1 H, s, NH), -1.96 (1 H, s, NH), 0.88-0.98 (9 H, m, 3 × C*H*₃), 1.33-1.55 (12 H, m, 6 × C*H*₂), 1.73-1.85 (6 H, m, 3 × C*H*₂), 2.20-2.45 (6 H, m, 3 × C*H*₂), 4.38-4.47 (2 H, m, C*H*₂), 4.60-4.67 (2 H, m, C*H*₂), 4.75 (2 H, m_{c}, C*H*₂), 5.30 (1 H, br s, COO*H*), 6.57 (2 H, s, *β*-H), 8.19 (2 H, d, *J* 8.1 Hz, Ar), 8.38 (1 H, d, *J* 4.5, *β-*H), 8.41 (2 H, d, *J* 8.1, Ar), 8.64 (1 H, d, *J* 4.9, *β*-H), 9.16-9.18 (2 H, m, *β*-H), 9.24 (1 H, d, *J* 5.0, *β*-H), 9.49 (1 H, d, *J* 5.0, *β*-H); *δ*_{C}(125 MHz; (CD₃)₂CO) 14.37 (CH₃), 14.44 (*C*H₃), 14.47 (*C*H₃), 23.40 (*C*H₂), 23.50 (*C*H₂), 23.54 (*C*H₂), 30.77 (*C*H₂), 31.04 (*C*H₂), 31.09 (*C*H₂), 32.64, (*C*H₂), 32.71 (*C*H₂), 32.74 (*C*H₂), 34.12 (*C*H₂), 34.49 (*C*H₂), 35.54 (*C*H₂), 37.24 (*C*H₂), 37.37 (*C*H₂), 39.06 (*C*H₂), 74.14 (*β*-C), 74.28 (*β-*C), 113.62 (*meso*-C), 113.70 (*meso-*C), 119.70 (*meso*-C), 122.71 (*meso*-C), 123.05 (*β-*C), 123.13 (*β*-C), 126.16 (*β*-C), 128.42 (*β*-C), 128.88 (Ar), 130.50 (*β*-C), 130.79 (*α-*C), 133.08 (*β*-C), 134.83 (Ar), 135.70 (*α*-C), 140.53 (*α*-C), 141.03 (*α*-C), 148.04 (Ar), 152.14 (*α*-C), 153.83 (*α*-C), 163.12 (*α*-C), 164.13 (*α*-C), 167.87 (CO₂H); *m*/*z* (ESI) 717.4399 ([M + H]⁺ C₄₅H₅₇N₄O₄⁺ requires 717.4374).

### 4.4 Reference Example Preparation of 5-(4-carboxyphenyl)-10,15,20-trihexyl-7,8-dihydroxy-7,8-chlorin

In a typical experiment, a solution of KOH (200 mg, 3.6 mmol) in methanol (1 ml) was added to a stirred solution of 5,10,15-trihexyl-17,18-dihydroxy-20-(4-methoxycarbonylphenyl)-17,18-chlorin (25 mg, 0.034 mmol) in THF (3 ml) and the reaction mixture was stirred for 2 d. Water (50 ml) and hydrochloric acid were then added until the pH was adjusted to 4-6. The aqueous layer was extracted with dichloromethane (2 x 100 ml) and the organic layer was separated, washed with water until neutral and dried over sodium sulfate. The solvent was evaporated and the title compound 5-(4-carboxyphenyl)-10,15,20-trihexyl-7,8-dihydroxy-7,8-chlorin was obtained after recrystallization from dichloromethane/aqueous methanol (22 mg, 90 %).

### 5-(4-Carboxyphenyl)-10,15,20-trihexyl-7,8-dihydroxy-7,8-chlorin

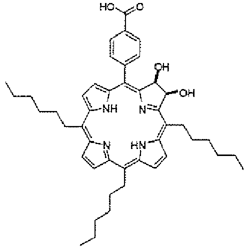

violet microcrystalline solid, mp 128-133 °C; *λ*ₘₐₓ(CH₃)₂CO)/nm 404 (*ε*/dm³ mol⁻¹ cm⁻¹ 152200), 428 (114600), 525 (13300), 550 (15900), 595 (6600) and 647 (18200); *δ*_{H}(500 MHz; (CD₃)₂CO) -1.78 (1 H, s, NH), -1.59 (1 H, s, NH), 0.87-0.96 (9 H, m, 3 x C*H*₃), 1.30-1.53 (12 H, m, 6 × C*H*₂), 1.68-1.80 (6 H, m, 3 × C*H*₂), 2.18-2.27 (1 H, m, HC*H*), 2.29-2.42 (5 H, m, 2 × C*H*₂, HC*H*), 4.32-4.82 (1 H, m, HC*H*), 4.54-4.60 (1 H, m, HC*H*), 4.61-4.72 (4 H, m, 2 × C*H*₂), 6.17 (1 H, d, *J* 7.1 *β*-H), 6.40 (1 H, d, *J* 7.1 *β*-H), 7.89-7.95 (1 H, br m, Ar), 8.11-8.16 (1 H, m, Ar), 8.17 (1 H, d, *J* 4.9, *β*-H), 8.22-8.36 (2 H, br m, Ar), 9.14 (1 H, d, *J* 5.0, *β*-H), 9.16 (1 H, d, *J* 4.7, *β*-H), 9.18 (1 H, d, *J* 4.7, *β*-H), 9.26 (1 H, d, *J* 4.9, *β*-H), 9.41 (1 H, d, *J* 5.0, *β*-H); *δ_{C}*(125 MHz; (CD₃)₂CO) 14.37 (*C*H₃), 14.40 (*C*H₃), 14.46 (*C*H₃), 23.38 (*C*H₂), 23.42 (*C*H₂), 23.51 (*C*H₂), 30.73 (*C*H₂), 30.79 (*C*H₂), 31.05 (*C*H₂), 32.62, (*C*H₂), 32.63 (*C*H₂), 32.72 (*C*H₂), 33.93 (*C*H₂), 35.48 (*C*H₂), 35.82 (*C*H₂), 37.16 (*C*H₂), 38.92 (*C*H₂), 39.12 (*C*H₂), 73.88 (*β*-C), 74.55 (*β*-C), 112.13 (*meso-*C), 113.27 (*meso-*C), 122.71 (*β*-C), 122.93 (*meso-*C), 122.96 (*meso-*C), 124.48 (*β*-C), 125.68 (*β*-C), 126.56 (*β*-C), 130.22 (*α*-C), 130.67 (*β*-C), 130.98 (*β*-C), 135.07 (*α*-C), 135.70 (*α*-C), 140.12 (*α*-C), 141.49 (*α*-C), 148.03 (Ar), 153.09 (*α*-C), 153.50 (*α*-C), 162.07 (*α*-C), 164.25 (*α*-C), 168.01 (*C*O₂H); *m*/*z* (ESI) 717.4356 ([M + H]⁺ C₄₅H₅₇N₄O₄⁺ requires 717.4374).

### Example 5

### Reference Example Preparation of porphyrins containing carbohydrate moieties

### 5 Preparation of 5,15-dihexyl-10,20-bis-(3-β-D-acetoglucosylphenyl)-porphyrin and 5,10-dihexyl-15,20-bis-(3-β-D-acetoglucosylphenyl)-porphyrin

In a typical experiment, dry dichloromethane (528 ml) was placed in a three-necked flask equipped with a magnetic stirrer and argon gas inlet. After pyrrole (528 *µ*l, 7.62 mmol), heptanal (560 *µ*l, 3.96 mmol) and tetraacetyl-*β*-D-glucopyranosyloxy-benzaldehyde (600 mg, 1.32 mmol) were added, the flask was shielded from ambient light and TFA (408 *µ*l, 5.28 mmol) was added and the reaction mixture was stirred at room temperature for 18 h. Then, DDQ (898 mg, 3.96 mmol) suspended in dry dichloromethane (30 ml) was added. After further stirring for 1 h, triethylamine (1 ml) was added. To remove polymeric by-products, the reaction mixture was filtered through silica gel. The solvent was evaporated and purification was achieved via repeated flash chromatography with hexane/ethylacetate 60:40 and dichloromethane/ethylacetate 90:10 as eluent, followed by recrystallization from dichloromethane/methanol The first band from the column contained 5,10,15,20-tetrahexyl-porphyrin (51 mg, 8 %), the second band 5,10,15-trihexyl-20-bis-(3-*β*-D-acetoglucosylphenyl)-porphyrin (145 mg, 11 %), the third band the title compound 5,15-dihexyl-10,20-bis-(3-*β*-D-acetoglucosylphenyl)-porphyrin (33 mg, 4 %) and the fourth band contained the title compound 5,10-dihexyl-15,20-bis-(3-*β*-D-acetoglucosylphenyl)-porphyrin (57 mg, 7 %).

### 5,15-Dihexyl-10,20-bis-(3-β-D-acetoglucosylphenyl)-porphyrin

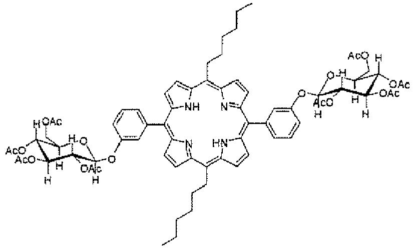

violet microcrystalline solid, *δ*_{H}(500 MHz; CDCl₃) -2.72 (2 H, s, NH), 0.91-0.96 (6 H, m, 2 × *C*H₃), 1.31-1.33 (6 H, m, Ac), 1.36-1.43 (4 H, m, 2 × C*H*₂), 1.48-1.56 (4 H, m, 2 × C*H*₂), 1.77-1.84 (4 H, m, 2 × C*H*₂), 1.98 (6 H, s, Ac), 2.04 (6 H, s, Ac), 2.11 (6 H, s, Ac), 2.49-2.55 (4 H, m, 2 × C*H*₂), 3.76-3.81 (2 H, m, H-5 'ose'), 4.03-4.07 (2 H, m, H-6 'ose') 4.14-4.18 (2 H, m, H-6 'ose'), 4.94-5.00 (4 H, m, 2 × C*H*₂), 5.15-5.20 (2 H, m, H-4 'ose'), 5.31-5.34 (2 H, m, H-2/3 'ose'), 5.35-5.37 (2 H, m, H-1 'ose'), 5.39-5.42 (2 H, m, H-2/3 'ose'), 7.43-7.46 (2 H, m, Ar), 7.66-7.69 (2 H, m, Ar), 7.84-7.87 (2 H, m, Ar), 7.92-7.95 (2 H, m, Ar), 8.89 (4 H, d, *J* 4.7, *β*-H), 9.43-9.46 (4 H, m, *β-*H); *δ*_{C}(125 MHz; CDCl₃) 14.23 (*C*H₃), 19.96 (CO*C*H₃), 20.60 (CO*C*H₃), 20.69 (CO*C*H₃), 20.81 (CO*C*H₃), 22.82 (*C*H₂), 30.31 (*C*H₂), 31.99 (*C*H₂), 35.42 (*C*H₂), 38.88 (*C*H₂), 61.99 (C-6 'ose'), 68.38 (C-4 'ose'), 71.40 (C-2/3 'ose'), 72.26 (C-5 'ose'), 72.89 (C-2/3 'ose'), 99.38 (C-1 'ose'), 116.71 (Ar), 118.10 (*meso-*C), 120.21 *(meso-C),* 122.77 (Ar), 127.67 (Ar), 128.07 (*β*-C), 129.94 (Ar), 131.70 (*β*-C), 144.31 (Ar), 155.34 (Ar), 169.44 (2 × *C*OCH₃), 170.30 (*C*OCH₃), 170.45 (*C*OCH₃); *m*/*z* (ESI) 1323.5 ([M + H]⁺ C₇₂H₈₃N₄O₂₀⁺ requires 1323.6).

### 5,10-Dihexyl-15,20-bis-(3-β-D-acetoglucosylphenyl)-porphyrin

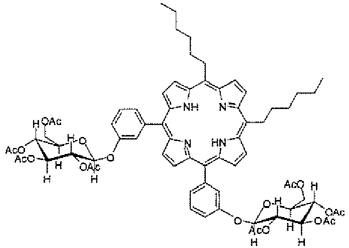

violet microcrystalline solid, *δ*_{H}(500 MHz; CDCl₃) -2.71 (2 H, s, NH), 0.95-0.98 (6 H, m, 2 × C*H*₃), 1.32-1.38 (6 H, m, Ac), 1.40-1.47 (4 H, m, 2 × C*H*₂), 1.52-1.59 (4 H, m, 2 × C*H*₂), 1.82-1.89 (4 H, m, 2 × C*H*₂), 1.98-2.01 (6 H, m, Ac), 2.04-2.05 (6 H, m, Ac), 2.10-2.12 (6 H, m, Ac), 2.52-2.61 (4 H, m, 2 × C*H*₂), 3.75-3.84 (2 H, m, H-5 'ose'), 4.02-4.07 (2 H, m, H-6 'ose') 4.14-4.21 (2 H, m, H-6 'ose'), 5.00-5.04 (4 H, m, 2 × C*H*₂), 5.15-5.21 (2 H, m, H-4 'ose'), 5.30-5.43 (6 H, m, H-1, H-2, H-3 'ose'), 7.41-7.45 (2 H, m, Ar), 7.63-7.70 (2 H, m, Ar), 7.82-7.95 (4 H, m, Ar), 8.76-8.79 (2 H, m, *β*-H), 8.87-8.91 (2 H, m, *β*-H), 9.45-9.49 (2 H, m, *β*-H), 9.59 (2 H, s, *β*-H); *δ*_{C}(125 MHz; CDCl₃) 14.25 (*C*H₃), 19.96 (CO*C*H₃), 20.61 (CO*C*H₃), 20.69 (CO*C*H₃), 20.80 (CO*C*H₃), 22.85 (*C*H₂), 30.41 (*C*H₂), 32.02 (*C*H₂), 35.84 (*C*H₂), 39.05 (*C*H₂), 62.01 (C-6 'ose'), 68.39 (C-4 'ose'), 71.39 (C-2/3 'ose'), 72.27 (C-5 'ose'), 72.89 (C-2/3 'ose'), 99.24 (C-1 'ose'), 116.52 (Ar), 117.75 (*meso*-C), 120.68 (*meso*-C), 122.79 (Ar), 129.95 (Ar), 129.94 (Ar), 143.97 (Ar), 155.45 (Ar), 169.45 (2 × *C*OCH₃), 170.31 (*C*OCH₃), 170.47 (*C*OCH₃); *m*/*z* (ESI) 1323.5 ([M + H]⁺ C₇₂H₈₃N₄O₂₀⁺ requires 1323.6).

### Example 6

### Cell tests of selected compounds in the HT 29 cell line

The photosensitzing activity was determined in the human colon adenocarcinoma cell line HT29. The HT29 cell lines were grown in DMEM (cc-pro GmbH) supplemented with 10 % heat-inactivated fetal calf serum (FCS, cc-pro GmbH), 1 % penicillin (10000 IU) and streptomycin (10000 *µ*g/ml, cc-pro GmbH). Cells were kept as a monolayer culture in a humidified incubator (5 % CO₂ in air at 37 °C.

A photosensitizer stock solution (2 mM) was performed in DMSO and was kept in the dark at 4 °C. Further dilution was performed in RPMI 1640 medium without phenol red supplemented with 10 % FCS to reach a final photosensitizer concentration of 2 or 10 *µ*M, respectively.

2 · 10⁴ cells/ml were seeded in micro plates (2 · 10⁵ cells/well). Cells were incubated with fresh medium (RPMI without phenol red) containing 10 % FCS with 2 or 10 *µ*M of the photosensitizer for 24 h before light exposure. Before photosensitization, cells were washed, incubated with RPMI without phenol red and 10 % FCS, then irridiated at room temperature with a 652 nm diode laser (Ceralas PDT 652, biolitec AG) at a fixed fluence rate of 100 mW/cm² (50 J/cm²). Following irradiation, cells were incubated in a humidified incubator (5 % CO₂ in air at 37 °C) for 24 h until cell viability assay.

The cell viability was assessed by the XTT assay. 500 mg XTT (sodium 3'-[phenylaminocarbonyl)-3,4-tetrazolium]-bis(4-methoxy-6-nitro)benzene sulfonic acid, Applichem GmbH) is dissolved in 500 ml PBS-Buffer (without Ca²⁺ and Mg²) and sterile filtered. Solution was stored in the dark at -20 °C until use. A sterile solution containing PMS (N-methyl dibenzopyrazine methyl sulfate, Applichem GmbH) was needed as an activation reagent for the XTT. 0.383 mg PMS was dissolved in 1 ml PBS-Buffer The solution should be stored frozen and should not be exposed to light. The XTT reagent solution was thawed in a 37 °C water bath and the activation solution (PMS) was added immediately prior to use. To prepare a reaction solution sufficient for one micro plate (96 wells), 0.1 ml activation solution (PMS) was given to 5 ml XTT reagent. The medium in the micro plate was exchanged with fresh RPMI without phenol red and 10 % FCS (100 *µ*l) prior adding 50 *µ*l XTT reaction solution per well. The micro plate was incubated for 2-3 hours at 37 °C and 5 % CO₂ until an orange dye is to be formed. The micro plate has been shaken gently to evenly distribute the dye in the wells.

The absorbance of the samples was measured with a spectrophotometer (Bio-Kinetics Reader EL312 e; Bio-Tek Instruments Inc.) at a wavelength of 490 nm. In order to measure reference absorbance (to measure non-specific readings) a wavelength of 630-690 nm was used.

The examples 6.1 to 6.3 illustrate the photodynamic activity (DT means dark toxicity and Laser means photo toxicity) of photosensitizers having an unsymmetrical substitution pattern. The three photosensitizers have an A₂B₂-substitution pattern of the *meso*-substituents, combining two polar and two nonpolar *meso*-substituents. Especially 5,15-bis-(4-carboxyphenyl)-10,20-dihexyl-7,8-dihydroxy-7,8-chlorin (compound according to the invention) exhibits a very strong photodynamic activity in the HT29 cell line, which is known to be very resistant against cell-toxic agents and PDT as well.

The examples 6.4 to 6.6 are included to illustrate, that chlorin photosensitizers which *do not have* an unsymmetrical substitution pattern *do not exhibit a promising photodynamic activity* in the cell experiments. The three photosensitizers in this case are based on an A₃B-substitution pattern of the *meso*-substituents. In fact, in example 6.6, where there are three hexyl chains and one benzoic ester group at the *meso*-positions the cell viability seems to increase (!) under irradiation (for a photosensitizer concentration of 10 µM, cf. below).

### 6.1 Cell test of 5,15-bis-(4-carboxyphenyl)-10,20-dihexyl-7,8-dihydroxy-7,8-chlorin

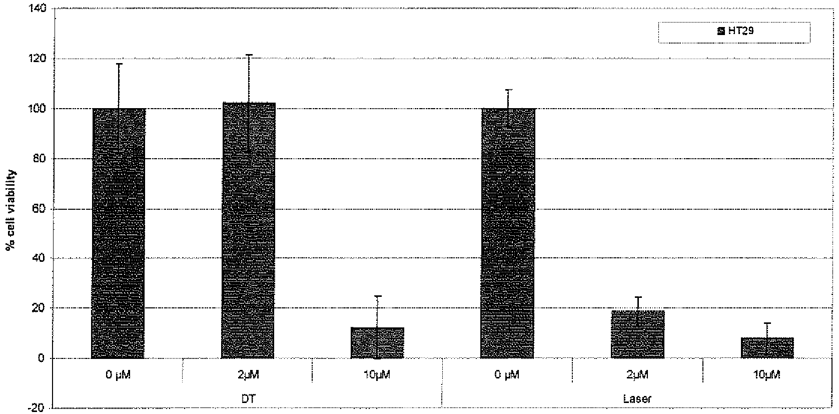

### 6.2 Cell test of 5,20-bis-(4-carboxyphenyl)-10,15-dihexyl-7,8-dihydroxy-7,8-chlorin

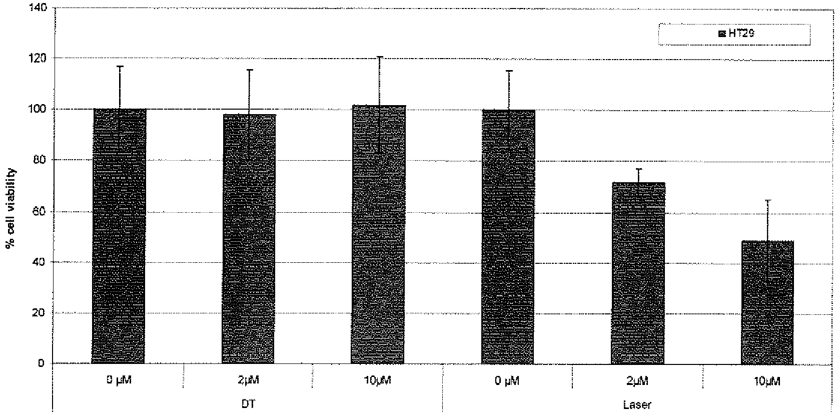

### 6.3 Cell test of 5,10-bis-(4-carboxyphenyl)-15,20-dihexyl-porphyrin

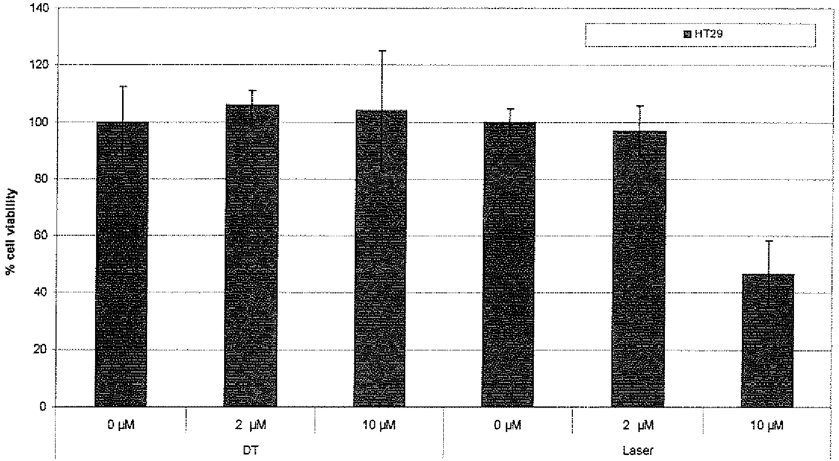

### 6.4 Cell test of 5-(4-carboxyphenyl)-10,15,20-trihexyl-7,8-dihydroxy-7,8-chlorin

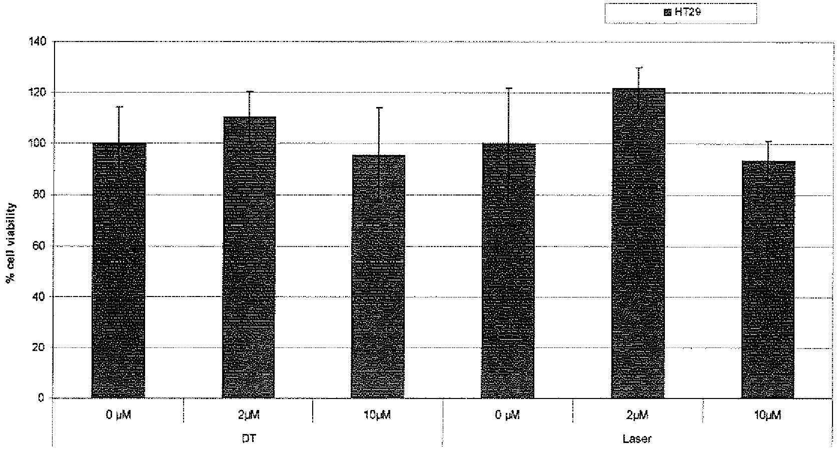

### 6.5 Cell test of 5-(4-carboxyphenyl)-10,15,20-trihexyl-17,18-dihydroxy-17,18-chlorin

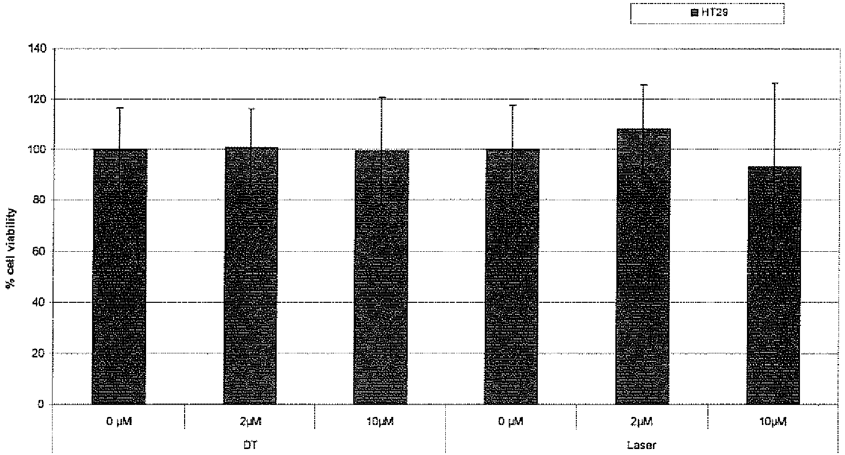

### 6.6 Cell test of 5,10,15-trihexyl-7,8-dihydroxy-20-(4-methoxycarbonyl-phenyl)-7,8-chlorin

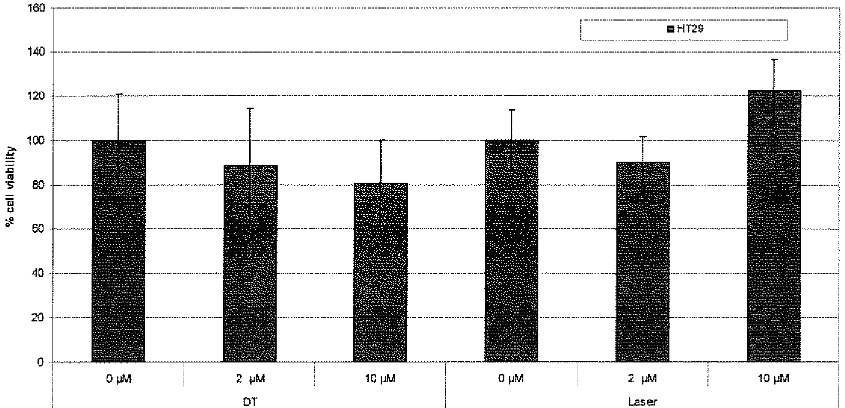

### Example 7

### Cell tests of selected compounds in a rabbit synoviocyte and a mouse macrophage cell line, HIG82 and J774A.1

The mouse monocytes-macrophages cell line J774A.1 and the rabbit synoviocyte cell line HIG-82 were grown in DMEM (cc-pro GmbH) supplemented with 10 % heat-inactivated fetal calf serum (FCS, cc-pro GmbH), 1 % penicillin (10000 IU) and streptomycin (10 000 *µ*g/ml, cc-pro GmbH). Cells were kept as a monolayer culture in a humidified incubator (5 % CO₂ in air at 37 °C).

A photosensitizer stock solution (2 mM) was performed in DMSO and was kept in the dark at 4 °C. Further dilution was performed in RPMI 1640 medium without phenol red supplemented with 10 % FCS to reach a final photosensitizer concentration of 2 or 10 *µ*M, respectively.

2 · 10⁴ cells/ml were seeded in micro plates (2 · 10⁵ cells/well). Cells were incubated with fresh medium (RPMI without phenol red) containing 10 % FCS with 2 or 10 *µ*M of the photosensitizer for 24 h before light exposure. Before photosensitization, cells were washed, incubated with RPMI without phenol red and 10 % FCS, then irridiated at room temperature with a 652 nm diode laser (Ceralas PDT 652, biolitec AG) at a fixed fluence rate of 100 mW/cm² (50 J/cm²). Following irradiation, cells were incubated in a humidified incubator (5 % CO₂ in air at 37 °C) for 24 h until cell viability assay.

The cell viability was assessed by the XTT assay. 500 mg XTT (sodium 3'-[phenylaminocarbonyl)-3,4-tetrazolium]-bis(4-methoxy-6-nitro)benzene sulfonic acid, Applichem GmbH) is dissolved in 500 ml PBS-Buffer (without Ca²⁺ and Mg²) and sterile filtered. Solution was stored in the dark at -20 °C until use. A sterile solution containing PMS (N-methyl dibenzopyrazine methyl sulfate, Applichem GmbH) was needed as an activation reagent for the XTT. 0.383 mg PMS was dissolved in 1 ml PBS-Buffer The solution should be stored frozen and should not be exposed to light. The XTT reagent solution was thawed in a 37 °C water bath and the activation solution (PMS) was added immediately prior to use. To prepare a reaction solution sufficient for one micro plate (96 wells), 0.1 ml activation solution (PMS) was given to 5 ml XTT reagent. The medium in the micro plate was exchanged with fresh RPMI without phenol red and 10 % FCS (100 *µ*l) prior adding 50 *µ*l XTT reaction solution per well. The micro plate was incubated for 2-3 hours at 37 °C and 5 % CO₂ until an orange dye is to be formed. The micro plate has been shaken gently to evenly distribute the dye in the wells.

The absorbance of the samples was measured with a spectrophotometer (Bio-Kinetics Reader EL312 e; Bio-Tek Instruments Inc.) at a wavelength of 490 nm. In order to measure reference absorbance (to measure non-specific readings) a wavelength of 630-690 nm was used.

The examples 7.1 to 7.4 illustrate the photodynamic activity of photosensitizers having an unsymmetrical substitution pattern against synoviocytes and macrophages, cell types which are especially relevant for the treatment of arthritis and similar inflammatory diseases. The four photosensitizers have the A₂B₂-substitution pattern of the *meso-*substituents, combining two polar and two nonpolar *meso*-substituents. Especially, 5,15-bis-(4-carboxyphenyl)-10,20-dihexyl-7,8-dihydroxy-7,8-chlorin (compound according to the invention) and 5,20-bis-(4-carboxyphenyl)-10,15-dihexyl-7,8-dihydroxy-7,8-chlorin (reference example) exhibit a very strong photodynamic activity in the two cell lines.

The example 7.5 is again included to illustrate, that chlorin photosensitizers which *do not have* an unsymmetrical substitution pattern *do not exhibit a promising photodynamic activity.*

### 7.1 Cell test of 5,15-bis-(4-carboxyphenyl)-10,20-dihexyl-7,8-dihydroxy-7,8- chlorin

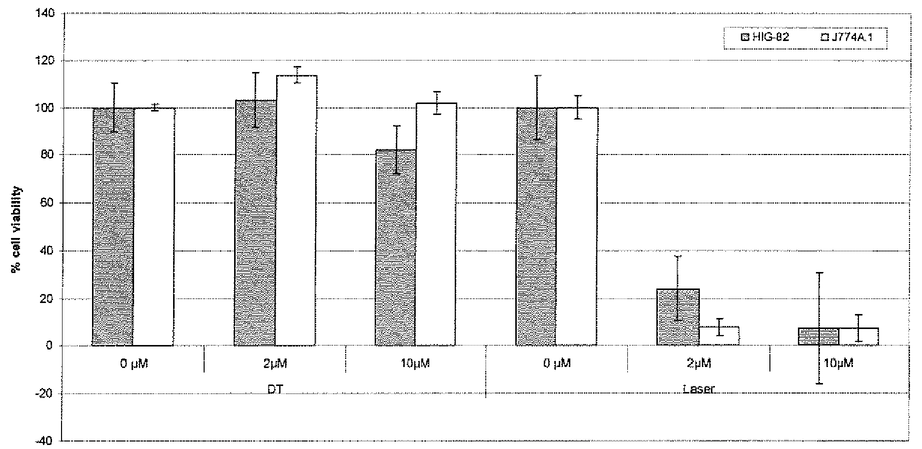

### 7.2 Cell test of 5,20-bis-(4-carboxyphenyl)-10,15-dihexyl-7,8-dihydroxy-7,8- chlorin

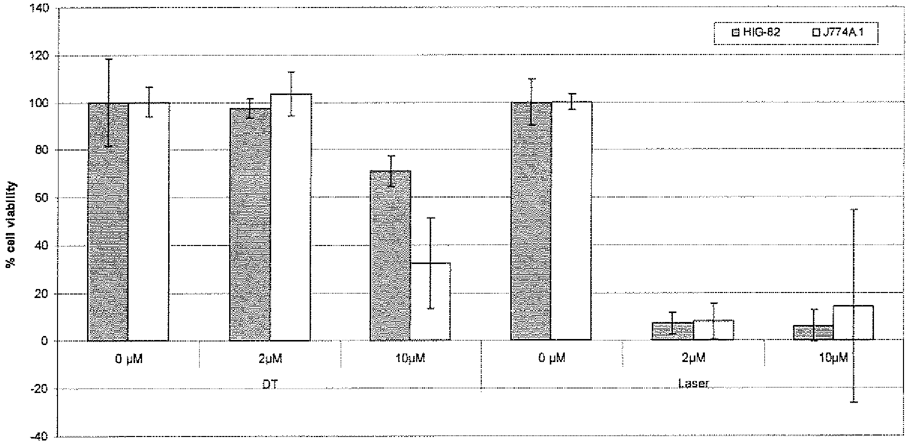

### 7.3 Cell test of 7,8-dihydroxy-5,10-bis-(3-hydroxyphenyl)-15,20-bis-(tridecyl)-7,8- chlorin

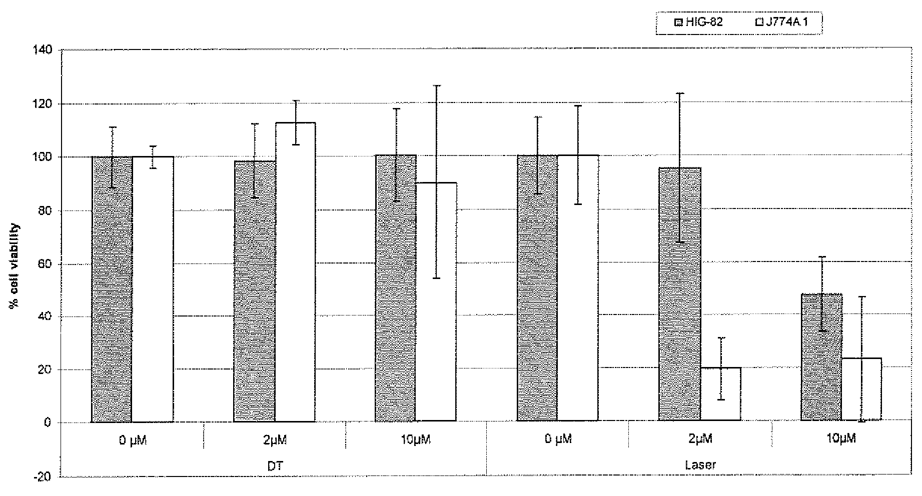

### 7.4 Cell test of 5,10-bis-(4-carboxyphenyl)-15,20-dihexyl-porphyrin

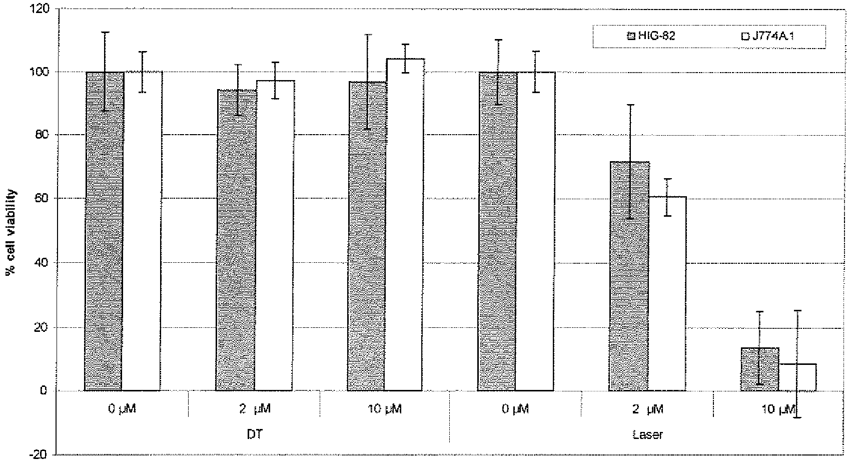

### 7.5 Cell test of 5,10,15-trihexyl-7,8-dihydroxy-20-(4-methoxycarbonyl-phenyl)- 7,8-chlorin

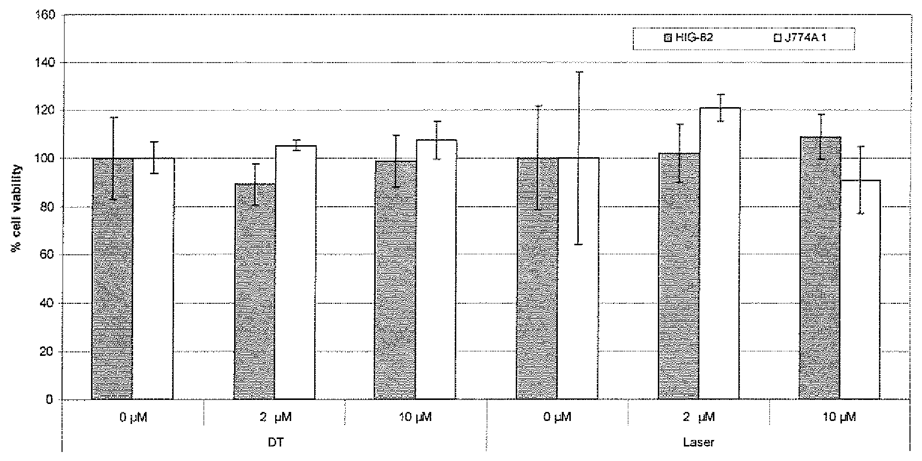

### Example 8

### Cell testing of a liposomal formulation of 5,15-bis-(4-carboxyphenyl)-10,20-dihexyl-7,8-dihydroxy-7,8-chlorin

The liposomal formulation of the photosensitizer 5,15-bis-(4-carboxyphenyl)-10,20-dihexyl-7,8-dihydroxy-7,8-chlorin was prepared in analogy to the procedure described in the US patent US 7,354,599 B2 by V. Albrecht, A. Fahr et al.

The tests with the liposomal formulation illustrate that the photosensitizers of the present invention may be formulated into liposomes for the purpose of e.g. a PDT or arthritis treatment without losing their photodynamic activity.

The liposomal formulation of the photosensitizer can be used to e.g. influence the pharmacokinetics of photosensitizer absorption and distribution and increase the bioavailability.

### 8.1 Cell test of a liposomal formulation of 5,15-bis-(4-carboxyphenyl)-10,20- dihexyl-7,8-dihydroxy-7,8-chlorin in the HT 29 cell line

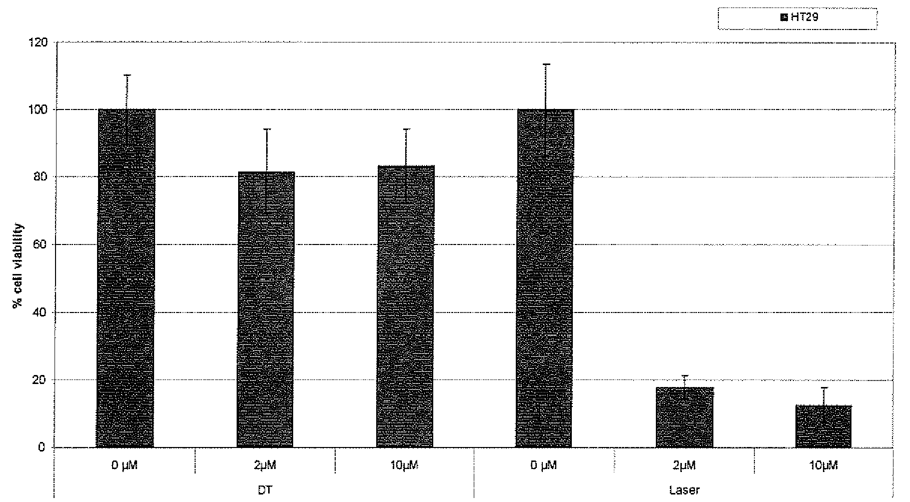

### 8.2 Cell test of a liposomal formulation of 5,15-bis-(4-carboxyphenyl)-10,20- dihexyl-7,8-dihydroxy-7,8-chlorin in the HIG82 and J774A.1 cell line

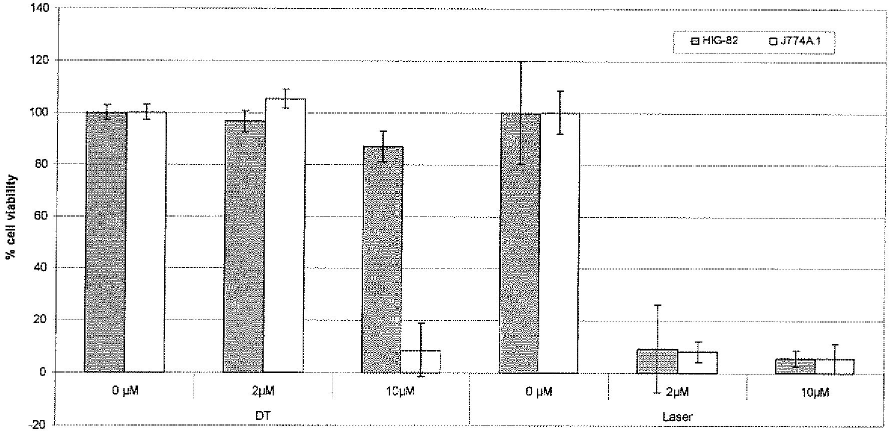

Having described preferred embodiments of the invention with reference to the accompanying examples, it is to be understood that the invention is not limited to the precise embodiments, and the scope of the invention is as defined in the appended claims.

## Claims

1. An unsymmetrically *meso*-substituted tetrapyrrolic compound comprising two polar and two nonpolar meso-substituents based specifically on the formula: 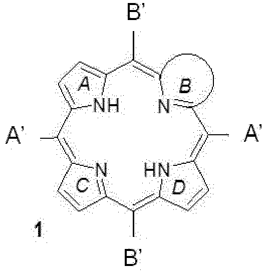
wherein the *meso*-substituents have an A'₂B'₂-substitution pattern;
wherein B is selected from the group consisting of: 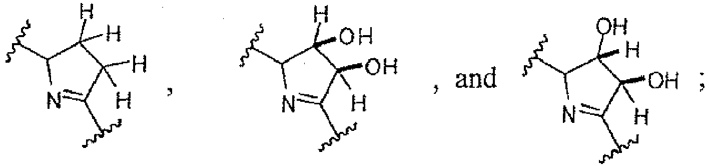
wherein A' is a unsubstituted alkyl group or fluoroalkyl group consisting of 4-15 carbon atoms; and
wherein B' is a phenyl group having the formula: 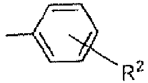
wherein R² is a substituent either in the *meta*- or *para*- position of the phenyl ring selected from the group consisting of OH, -COOH, -COOCH₃, -NH₂, -COOX, -NHX, -OX, -NH-Y-COOH and CO-Y-NH₂;
wherein:
X is a polyethyleneglycol-residue with (CH₂CH₂O)ₙCH₃ with n = 1-30 or a carbohydrate moiety and
Y is a peptide or oligopeptide wherein n = 1 - 30.

2. The tetrapyrrolic compound according to claim 1 based on formula **1** and having the formula: 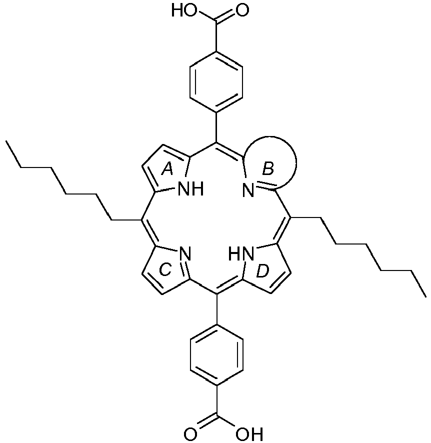 wherein B is selected from the group consisting of: 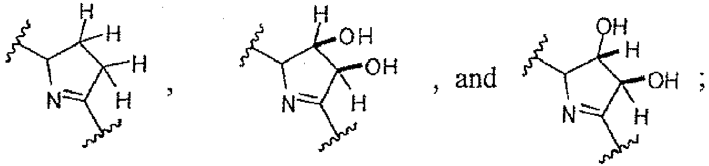 or a pharmaceutically acceptable derivative thereof.

3. The compound according to claim 1 or 2 or a pharmaceutically acceptable derivative thereof for use in photodynamic therapy.

4. Use of the compound of claim 1 or 2 or a pharmaceutically acceptable derivative thereof for the preparation of a pharmaceutical composition for photodynamic therapy.

5. The compound of claim 1 or 2 or a pharmaceutically acceptable derivative thereof for use in photodynamic therapy of tumors, dermatological disorders, viral or bacterial infections, ophthalmological disorders or urological disorders.

6. The compound of claim 1 or 2 or a pharmaceutically acceptable derivative thereof for use in the diagnosis or photodynamic therapy of arthritis and similar inflammatory diseases.

7. A pharmaceutical composition comprising a compound according to claim 1 or 2 or a pharmaceutically acceptable derivative thereof as an active ingredient.

8. A pharmaceutical composition in which a compound according to claim 1 or 2 or a pharmaceutically acceptable derivative thereof is conjugated to a targeting agent.

9. The pharmaceutical composition according to claim 8 in which an antibody or a fragment of an antibody is the targeting agent.

10. The pharmaceutical composition according to claim 8 or 9 in which said pharmaceutical composition is a liposomal formulation.

11. A pharmaceutical composition containing a compound according to claim 1 or 2 in which said pharmaceutical composition is a liposomal formulation.

## Patentansprüche

1. Unsymmetrisch *meso*-substituierte Tetrapyrrol-Verbindung mit zwei polaren und zwei nichtpolaren *meso*-Substituenten, die speziell auf der Formel beruhen: 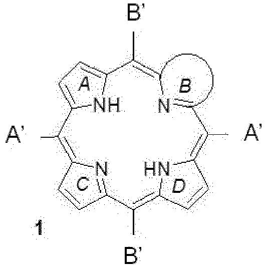
bei der die *meso*-Substituenten ein A'₂B'₂-Substitutionsmuster haben;
wobei B aus der Gruppe ausgewählt ist, die aus 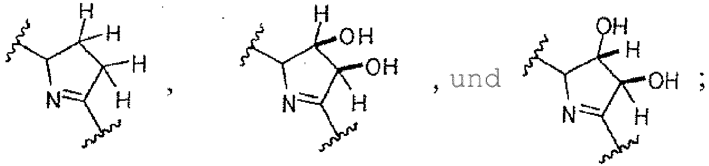 besteht,
wobei A' eine unsubstituierte Alkylgruppe oder Fluoroalkylgruppe ist, die aus 4 bis 15 Kohlenstoffatomen besteht; und
wobei B' eine Phenylgruppe mit der Formel ist: 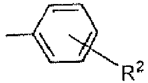 wobei R² ein Substituent entweder in der *meta-* oder *para-*Position des Phenylrings ist und aus der Gruppe ausgewählt ist, die aus OH, -COOH, -COOCH₃, -NH₂, -COOX, -NHX, -OX,-NH-Y-COOH und CO-Y-NH₂;
wobei:
X ein Polyethylenglykolrest mit (CH₂CH₂O)ₙCH₃ mit n = 1-30 oder ein Kohlenhydratrest ist, und
Y ein Peptid oder Oligopeptid ist, wobei n = 1 - 30 ist.

2. Tetrapyrrol-Verbindung nach Anspruch 1 auf der Grundlage der Formel **1** und mit der Formel: 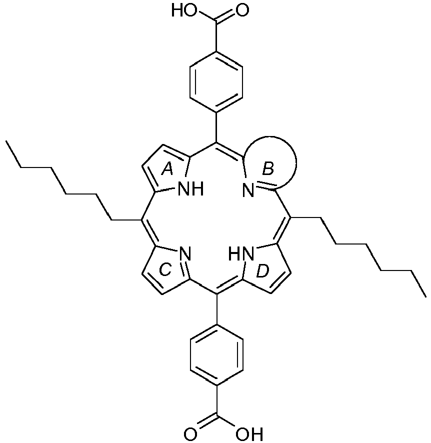 wobei B aus der Gruppe ausgewählt ist, die aus 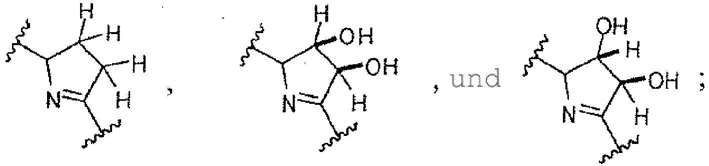 besteht,
oder ein pharmazeutisch akzeptierbares Derivat hiervon.

3. Verbindung nach Anspruch 1 oder 2 oder ein pharmazeutisch akzeptierbares Derivat hiervon für die Verwendung in der photodynamischen Therapie.

4. Verwendung der Verbindung nach Anspruch 1 oder 2 oder ein pharmazeutisch akzeptierbares Derivat hiervon für die Zubereitung einer pharmazeutischen Zusammensetzung für die photodynamische Therapie.

5. Verbindung nach Anspruch 1 oder 2 oder ein pharmazeutisch akzeptierbares Derivat hiervon für die Verwendung in der photodynamischen Therapie von Tumoren, dermatologischen Erkrankungen, viralen oder bakteriellen Infekten, ophthalmologischen Erkrankungen oder urologischen Erkrankungen.

6. Verbindung nach Anspruch 1 oder 2 oder ein pharmazeutisch akzeptierbares Derivat hiervon für die Verwendung in der Diagnose oder der photodynamischen Therapie von Arthritis oder ähnlichen entzündlichen Krankheiten.

7. Pharmazeutische Zusammensetzung, die eine Verbindung nach Anspruch 1 oder 2 oder ein pharmazeutisch akzeptierbares Derivat hiervon als Wirkstoff enthält.

8. Pharmazeutische Zusammensetzung, in der eine Verbindung nach Anspruch 1 oder 2 oder ein pharmazeutisch akzeptierbares Derivat hiervon mit einem zielorientierten Wirkstoff verbunden ist.

9. Pharmazeutische Zusammensetzung nach Anspruch 8, in der der zielorientierte Wirkstoff ein Antikörper oder ein Fragment eines Antikörpers ist.

10. Pharmazeutische Zusammensetzung nach Anspruch 8 oder 9, bei der die pharmazeutische Zusammensetzung eine liposomale Formulierung ist.

11. Pharmazeutische Zusammensetzung mit einer Verbindung nach Anspruch 1 oder 2, bei der die pharmazeutische Zusammensetzung eine liposomale Formulierung ist.

## Revendications

1. Un composé tétrapyrrolique *méso*-substitué asymétriquement contenant deux méso-substituants polaires et non polaires basés spécifiquement sur la formule : 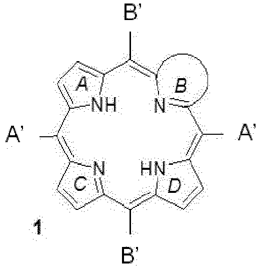
où les *méso*-substituants possèdent un motif de substitution A'₂B'₂,
où B est sélectionné dans le groupe se composant de : 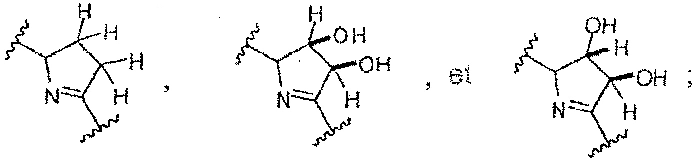
où A' est un groupe alkyle non substitué ou un groupe fluoroalkyle se composant de 4 à 15 atomes de carbone, et
où B' est un groupe phényle possédant la formule : 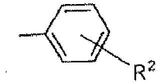 où R² est un substituant soit dans la position *méta-* ou *para-* du cycle phényle sélectionné dans le groupe se composant de OH, -COOH, -COOCH₃, -NH₂, -COOX,-NHX, -OX, -NH-Y-COOH et CO-Y-NH₂,
où :
X est un résidu polyéthylène glycol avec (CH₂CH₂O)ₙCH₃ avec n = 1-30 ou une fraction glucidique, et
Y est un peptide ou un oligopeptide où n = 1-30.

2. Le composé tétrapyrrolique selon la Revendication 1 basé sur la formule **1** et possédant la formule : 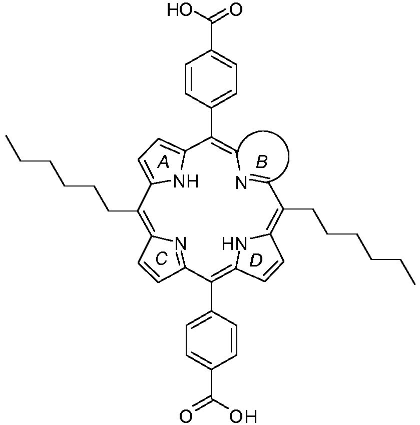 où B est sélectionné dans le groupe se composant de : 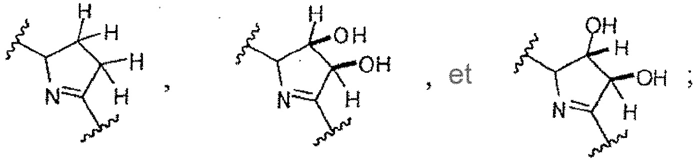 ou un dérivé pharmaceutiquement acceptable de celui-ci.

3. Le composé tétrapyrrolique selon la Revendication 1 ou 2 ou un dérivé pharmaceutiquement acceptable de celui-ci destiné à une utilisation en thérapie photodynamique.

4. L'utilisation du composé selon la Revendication 1 ou 2 ou un dérivé pharmaceutiquement acceptable de celui-ci pour la préparation d'une composition pharmaceutique destinée à une thérapie photodynamique.

5. Le composé selon la Revendication 1 ou 2 ou un dérivé pharmaceutiquement acceptable de celui-ci destiné à une utilisation en thérapie photodynamique de tumeurs, troubles dermatologiques, infections virales ou bactériennes, troubles ophtalmologiques ou troubles urologiques.

6. Le composé selon la Revendication 1 ou 2 ou un dérivé pharmaceutiquement acceptable de celui-ci destiné à une utilisation dans le diagnostic ou une thérapie photodynamique de l'arthrite et de maladies inflammatoires similaires.

7. Une composition pharmaceutique contenant un composé selon la Revendication 1 ou 2 ou un dérivé pharmaceutiquement acceptable de celui-ci en tant qu'ingrédient actif.

8. Une composition pharmaceutique dans laquelle un composé selon la Revendication 1 ou 2 ou un dérivé pharmaceutiquement acceptable de celui-ci est conjugué à un agent de ciblage.

9. La composition pharmaceutique selon la Revendication 8 dans laquelle un anticorps ou un fragment d'un anticorps est l'agent de ciblage.

10. La composition pharmaceutique selon la Revendication 8 ou 9 dans laquelle ladite composition pharmaceutique est une formulation liposomale.

11. Une composition pharmaceutique contenant un composé selon la Revendication 1 ou 2 dans laquelle ladite composition pharmaceutique est une formulation liposomale.
